# EUROPEAN PATENT APPLICATION

(11) **EP 4 663 139 A1**
(43) Date of publication of application: **17.12.2025**
(21) Application number: 24183342.5
(22) Date of filing: 20.06.2024
(51) Int. Cl.: A61B 17/70

(54) **A CLUSTER DEROTATION ASSEMBLY**

(30) Priority: 10.06.2024 US 202418738845
(71) Applicant: OrthoPediatrics Corp., Warsaw, IN 46582 (US)
(72) Inventor: GIBBS, Collin B., Columbia City, Indiana, 46725 (US); PRYGOSKI, Matthew Philip, North Liberty, Indiana, 46554 (US); DETLEFSEN, Richard, Warsaw, Indiana, 46580 (US); FOX, Camden L., Fort Wayne, Indiana, 46804 (US); LUBENSKY, Scott Alan, Warsaw, Indiana, 46582 (US); DANIELS, David W., Winona Lake, Indiana, 46590 (US)
(74) Representative: Patentanwälte Bressel und Partner mbB

(57) **Abstract**

In a cluster derotation assembly, a tube clip for joining a locking tube to a link member. The tube clip has a first clip arm and a second clip arm joined to a threaded shaft. One or both of the first clip arm and the second clip arm has an inner surface configured to engage with a complementary surface of a locking tube.

## Description

### BACKGROUND

Many individuals experience spinal column anomalies, such as scoliosis. Such anomalies may be corrected if manipulated, or "derotated," substantially as a whole into a desired configuration. To achieve such an objective, force must be applied safely to all to-be-derotated vertebrae, and the forces necessary to reconfigure all, or at least a substantial portion of the spinal column must be dispersed throughout the affected spinal segments or regions. In some instances, vertebral derotation can achieve three-dimensional correction of spinal deformities and reverse the torsional asymmetry induced by scoliosis.

### BRIEF DESCRIPTION OF THE DRAWINGS

It is believed that certain embodiments will be better understood from the following description taken in conjunction with the accompanying drawings, in which like references indicate similar elements and in which:
FIG. 1 depicts an apparatus being used in a medical procedure of spinal correction.
FIG. 2 depicts an example cluster derotation assembly affixed to two locking tubes.
FIG. 3 depicts an enlarged image of a portion of a cluster derotation assembly affixed to a locking tube.
FIG. 4 depicts an example locking tool.
FIG. 5 is a cross-sectional view of the Section 5-5 of FIG. 4.
FIG. 6 is a top view of an example cluster derotation assembly.
FIG. 7 is an elevation view of an example link member.
FIG. 8 is an elevation view of an example wing nut.
FIG. 9 is an elevation view of an example wing nut.
FIG. 10 is a cross-sectional view of the Section 10-10 of FIG. 6.
FIG. 11 depicts an example tube clip.
FIG. 12 depicts an example tube clip.
FIG. 13 depicts an example tube clip.
FIG. 14 depicts an example tube clip.
FIG. 15 depicts an example tube clip locked onto a locking tube.
FIG. 16 depicts an example cluster derotation assembly affixed to a locking tube.
FIG. 17 is a cross-sectional view of the Section 17-17 of FIG. 16.
FIG. 18 depicts an example cluster derotation assembly affixed to a locking tube.
FIG. 19 is a cross-sectional view of the Section 19-19 of FIG. 18.
FIG. 20 depicts an example cluster derotation assembly affixed to a locking tube.
FIG. 21 depicts an enlarged perspective image of a portion of a cluster derotation assembly affixed to a locking tube.
FIG. 22 depicts an elevation view of a portion of a cluster derotation assembly affixed to a locking tube.
FIG. 23 depicts an example cluster derotation assembly affixed to a locking tube.
FIG. 24 depicts an example cluster derotation assembly affixed to two locking tubes.
FIG. 25 is a cross-sectional view of the Section 25-25 of FIG. 24.
FIG. 25A is an enlarged view of a tube clip of FIG. 25.
FIG. 26 depicts a top view of an example cluster derotation assembly.
FIG. 27 depicts a partially exploded view of an example cluster derotation assembly.
FIG. 28 is a cross-sectional view of the Section 28-28 of FIG. 26.

### DETAILED DESCRIPTION

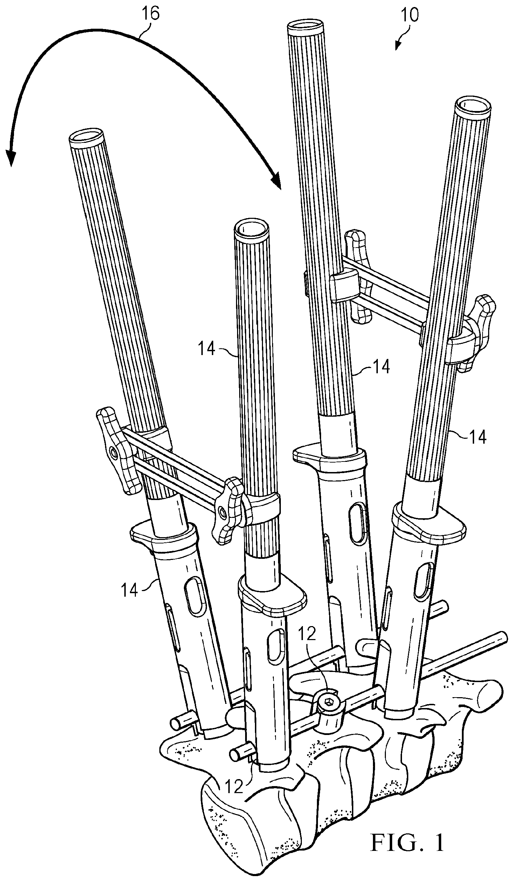

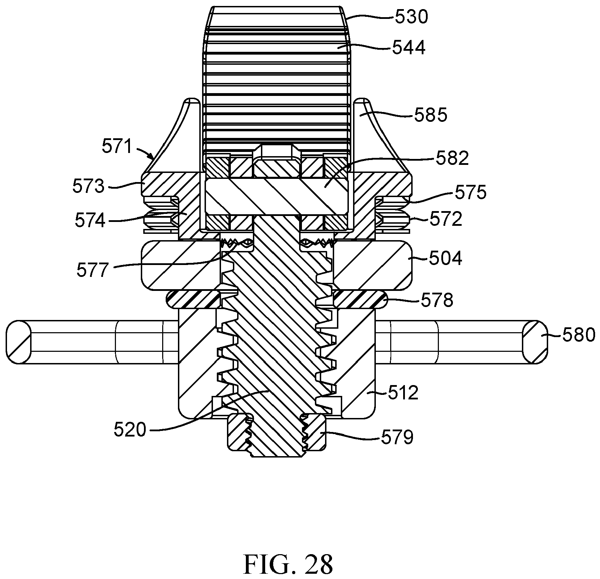

Various non-limiting embodiments of the present disclosure will now be described to provide an overall understanding of the principles of the structure, function, and use of the apparatus, systems and methods disclosed. One or more examples of these non-limiting embodiments are illustrated in the selected examples disclosed and described in detail with reference made to FIGS. 1 - 28 in the accompanying drawings. Those of ordinary skill in the art will understand that apparatus, systems and methods specifically described herein and illustrated in the accompanying drawings are non-limiting embodiments. The features illustrated or described in connection with one non-limiting embodiment may be combined with the features of other non-limiting embodiments. Such modifications and variations are intended to be included within the scope of the present disclosure.

The apparatus, systems and methods disclosed herein are described in detail by way of examples and with reference to the figures. The examples discussed herein are examples only and are provided to assist in the explanation of the apparatus, systems and methods described herein. None of the features or components shown in the drawings or discussed below should be taken as mandatory for any specific implementation of any of these apparatus, systems or methods unless specifically designated as mandatory. In this disclosure, any identification of specific techniques, arrangements, etc. are either related to a specific example presented or are merely a general description of such a technique, arrangement, etc. Identifications of specific details or examples are not intended to be, and should not be, construed as mandatory or limiting unless specifically designated as such. Any failure to specifically describe a combination or sub-combination of components should not be understood as an indication that any combination or sub-combination is not possible.

It will be appreciated that modifications to disclosed and described examples, arrangements, configurations, components, elements, apparatuses, devices, systems, methods, etc. can be made and may be desired for a specific application. Also, for any methods described, regardless of whether the method is described in conjunction with a flow diagram, it should be understood that unless otherwise specified or required by context, any explicit or implicit ordering of steps performed in the execution of a method does not imply that those steps must be performed in the order presented but instead may be performed in a different order or in parallel.

The apparatus, system, and method of the present disclosure relates to and facilitates application of forces to multiple spinal column segments, to achieve an over-all spinal column correction. As depicted in FIG. 1, a spinal column correction system and method 10 utilizes bone anchors such as bone screws, referred to herein as pedicle screws 12. Pedicle screws 12 are implanted in the pedicle region(s) of vertebrae to be derotated. Further, as shown in FIG. 1, a system and method of spinal column corrections uses shafts or similar pedicle screw engagement members, referred to herein as locking tubes 14. Locking tubes 14 can be secured, or locked, onto the head of pedicle screws 12 and manipulated by surgeons in various directions to facilitate movement of vertebra. In particular, spinal derotation can be facilitated by movement of the locking tubes of multiple vertebra in a suitable direction, as depicted, for example, by arrow 16.

The apparatus, system, and method of the present disclosure includes a pedicle screw cluster derotation assembly. In some embodiments, this tool includes a plurality of locking tubes in a linked array, such that the cluster derotation assembly facilitates engagement of the heads of a number of implanted pedicle screws which will have been implanted in adjacent vertebrae to which derotational or balancing forces are to be applied during a spinal column derotation and alignment. The engagement between the pedicle screw cluster derotation assembly and the individual pedicle screws is such that, as manipulative forces, for example, are applied to the pedicle screw cluster derotation assembly, forces are transferred and dispersed simultaneously among the engaged vertebrae, without (or with reduced) unwanted and detrimental forces applied to the individual pedicle screws. Therefore, in some embodiments, a practitioner may, in a single motion, simultaneously and safely derotate multiple vertebrae of an affected spinal segment (as well as likewise apply balancing forces to other group(s) of vertebrae which are lateral to the effected segment(s).

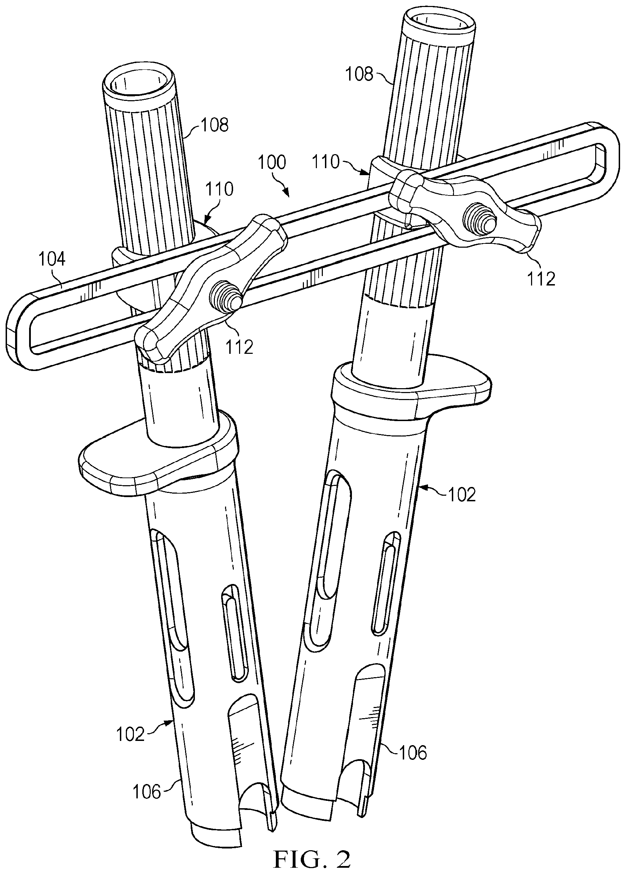

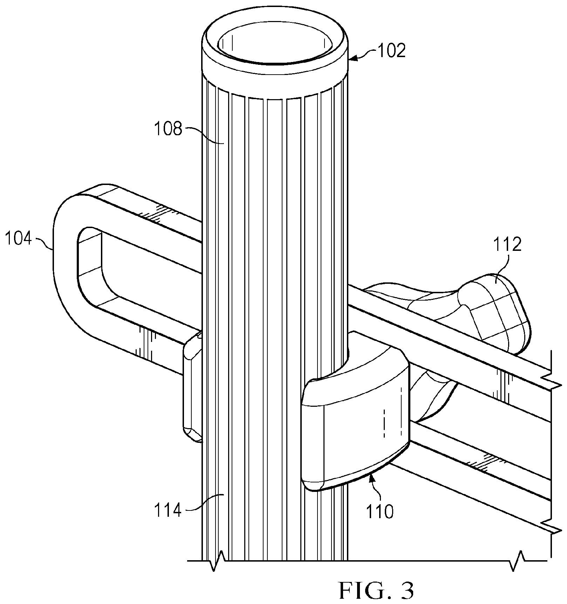

Referring now to FIGS. 2 and 3, there is shown an example system of a representative cluster derotation assembly 100. As depicted in FIG. 2, the cluster derotation assembly 100 is configured to join to a plurality of locking tubes 102, by a link member 104 that secures each of the plurality of locking tubes 102 in a rigid configuration such that they together act in unison during use (e.g., locking tubes 102 are fixed, for example, axially fixed, relative to each other). Each locking tube has a distal end portion 106 being configured to securely join to a bone anchor, and a proximal end portion 108, to which can be joined the link member 104. A plurality of tube clips 110, at least one for each locking tube 102, extend through the link member 104 to the locking tube 102, at or near the proximal end portion 108. Each tube clip 110 is secured by the action of a wing nut 112 being threaded and tightened onto a portion of the tube clip 110. As described in more detail below, a portion of the locking tube 102 may include a plurality of externally disposed axially-aligned grooves, ridges, flutes, or combinations thereof ("axially-aligned grooves"). Axially-aligned, as used herein, means aligned with the longitudinal axis and/or parallel with a longitudinal axis of the locking tube. The axially-aligned grooves 114 function to provide a surface or surfaces by which the axial rotation of the locking tube during use is minimized or eliminated.

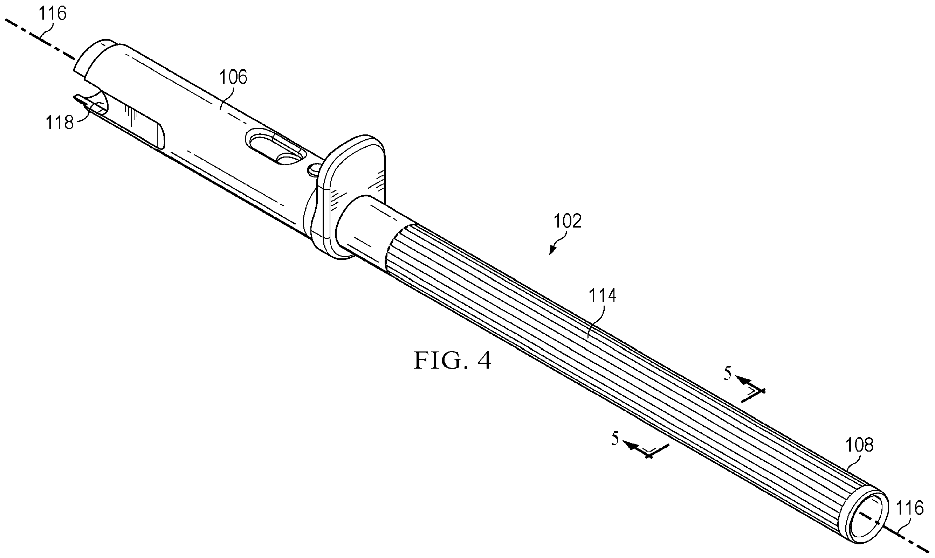

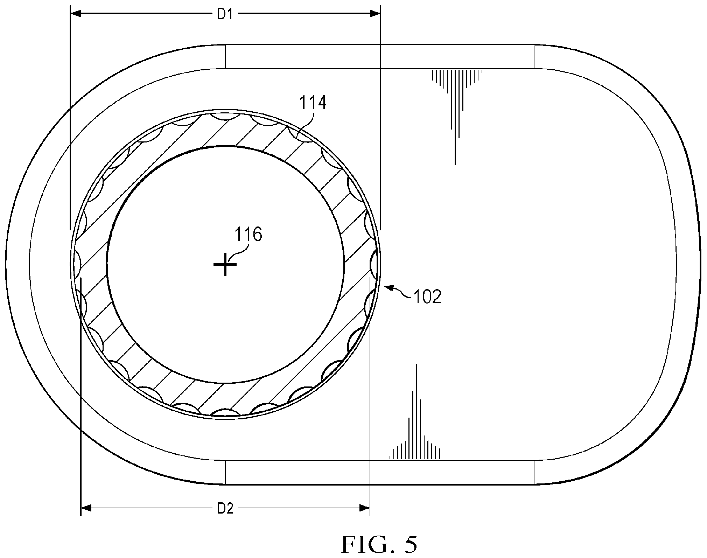

Referring now to FIGS. 4 and 5, there is depicted a locking tube 102. Each locking tube is generally cylindrically shaped and extends a suitable length about a central axis 116 (e.g., longitudinal axis). The locking tube 102 includes at a distal end portion 106 containing a collet 118 configured to slide over and reversibly engage the head segment of a pedicle screw such that, as locking tube 102 is moved while the collet 118 is clamped, tightened, or otherwise securely engaged with the head segment, manipulative forces are transferred to the pedicle screw and, in turn, to the vertebra in which such pedicle screw is implanted. As discussed above, each locking tube, in some embodiments and as shown herein, has a plurality of externally disposed, axially-aligned grooves 114. The number, size, and placement of the axially-aligned grooves 114 can be varied depending on other design considerations. As will be appreciated from the disclosure below, the number, size, and placement of the axially-aligned grooves 114 is determined and implemented in conjunction with the size, shape, and design of the tube clip 110. In an embodiment, there can be from about 12 to about 36 axially-aligned grooves 114. In the illustrated embodiment, there are 24 axially-aligned grooves. As depicted in FIG. 5, the tops (e.g., peaks) of the grooves define a maximum diameter D1 of the grooved portion of the locking tube 102. Likewise, for uniformly grooved locking tubes the valleys of the grooves define grooved diameter D2 of the grooved portion of the locking tube. As will be described more fully below, in operation a portion of a tube clip 110 will be operationally adjacent the locking tube at the maximum diameter D 1, while one or more portions of the tube clip 110 can be operationally disposed inside of the maximum diameter D1 and can be operationally adjacent or near the grooved diameter D2.

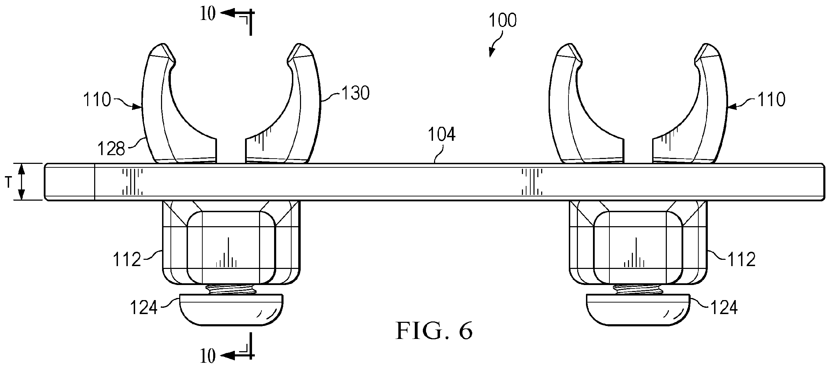

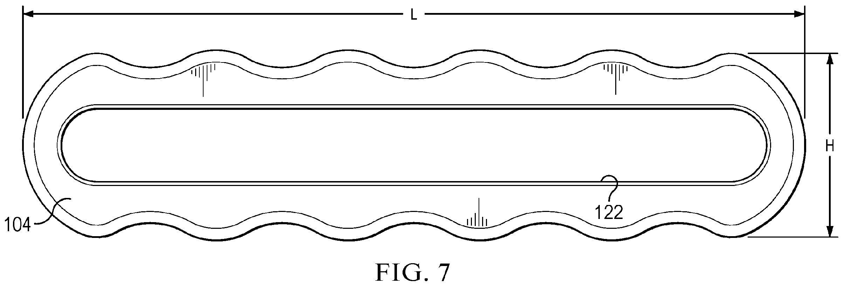

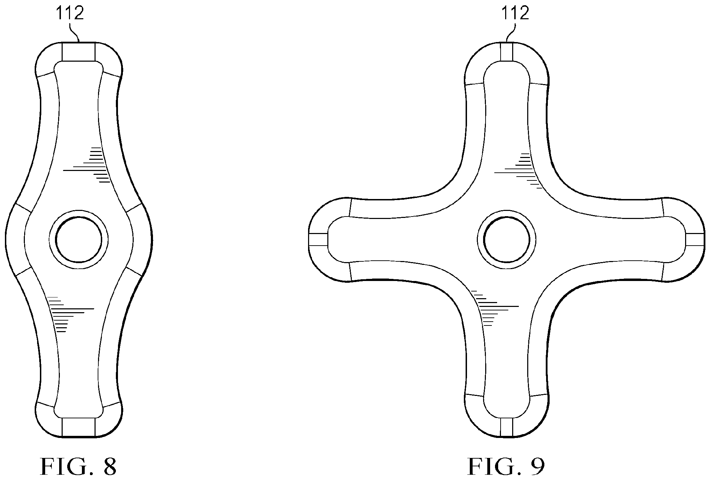

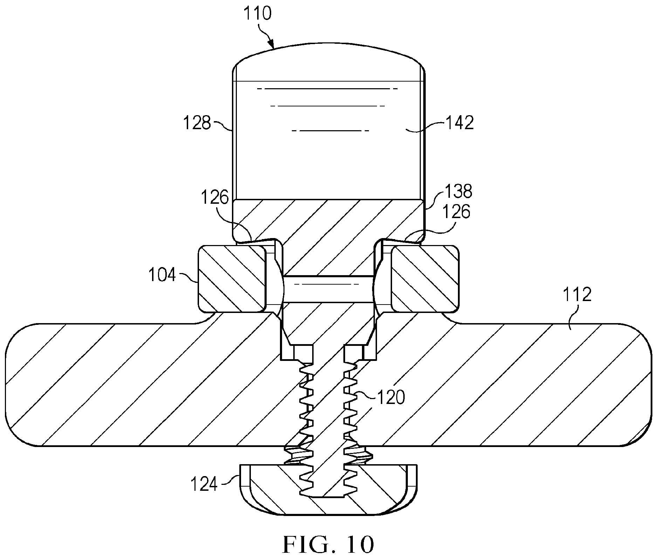

Referring now to FIG. 6 there is depicted a an embodiment of a cluster derotation assembly 100 configured to join to two locking tubes such as, for example, locking tube 102. In general, more than two locking tubes can be joined by the cluster derotation assembly 100. As an example, two tube clips 110 are shown mounted on the link member 104, and each is secured in place by a wing nut 112 threaded on to a threaded shaft 120 of the tube clip 110 (as shown in FIG. 10). Other securement mechanisms can be used, such as push-button securement, or a quickrelease cam lock mechanism, each of which can minimize the time and effort to secure the link member to a locking tube. A representative link member 104 is shown in FIG. 7. Other representative link members are described below. The link member can be a relatively rigid component having a thickness T, a length L, and a height H sufficient to provide the benefit of being a relatively rigid connector. In some embodiments, the link member 104 may also be lengthened to allow connection to more locking tubes (e.g., connection to 3, 4, 5, 6, 7 locking tubes, etc.). The link member can be a rigid body having defined therein an open slot 122 through which the threaded shaft 120 of the tube clip traverses. FIGS. 8 and 9 depict representative wing nuts 112 that can be utilized in the cluster derotation assembly 100.

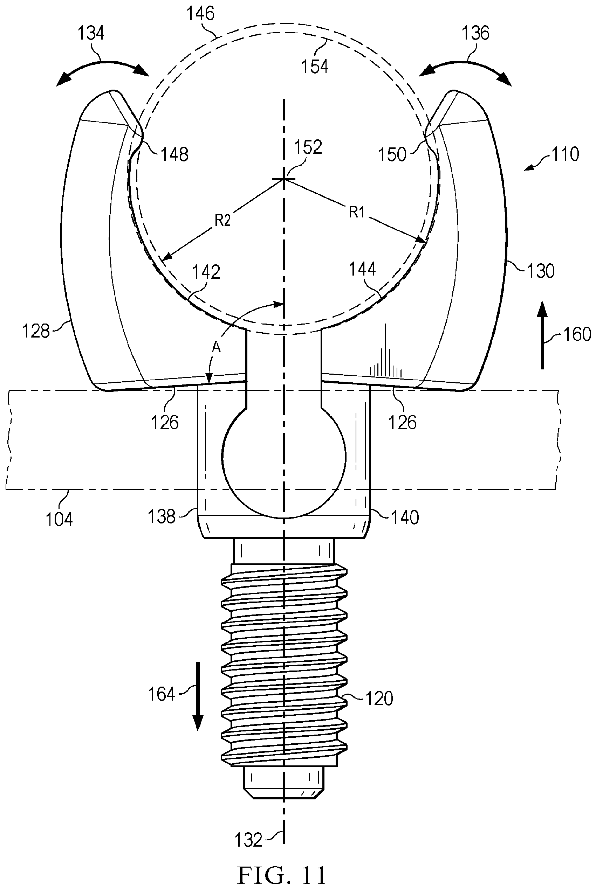

FIG. 10 is a cross-sectional view of Section 10-10 of FIG. 6, and FIG. 11 is a side view of a tube clip 110. As depicted in FIGS. 10 and 11, the threaded shaft 120 is sized to have a diameter less than the width of the open slot 122 of the link member 104, such that in use it extends through the link member 104. The wing nut 112 threads onto the threaded shaft 120, and a shaft cap 124 can be secured to the end of the threaded shaft 120 to prevent the wing nut 112 from threading off the end of the threaded shaft 120.

When the wing nut 112 is tightened onto the threaded shaft 120, the tube clip 110 is prevented from being drawn through the open slot 122 by respective shoulder members 126 at the base of a first clip arm 128 and a second clip arm 130. The shoulder members 126 are sized to extend onto and contact the link member 104 during use. The first clip arm 128 and the second clip arm 130 are joined to the threaded shaft 120 in a cantilevered configurations, such that each of the first clip arm 128 and the second clip arm 130 can be urged inwardly toward a threaded shaft axis 132, or outwardly away from the threaded shaft axis 132, as indicated by arrows 134 and 136, respectively. Thus, the first clip arm 128 can urged toward the threaded shaft axis 132 by bending about a first hinge portion 138 at the juncture of the first clip arm 128 and the threaded shaft 120. Likewise, the second clip arm 130 can urged toward the threaded shaft axis 132 by bending about a second hinge portion 140 at the juncture of the second clip arm 130 and the threaded shaft 120.

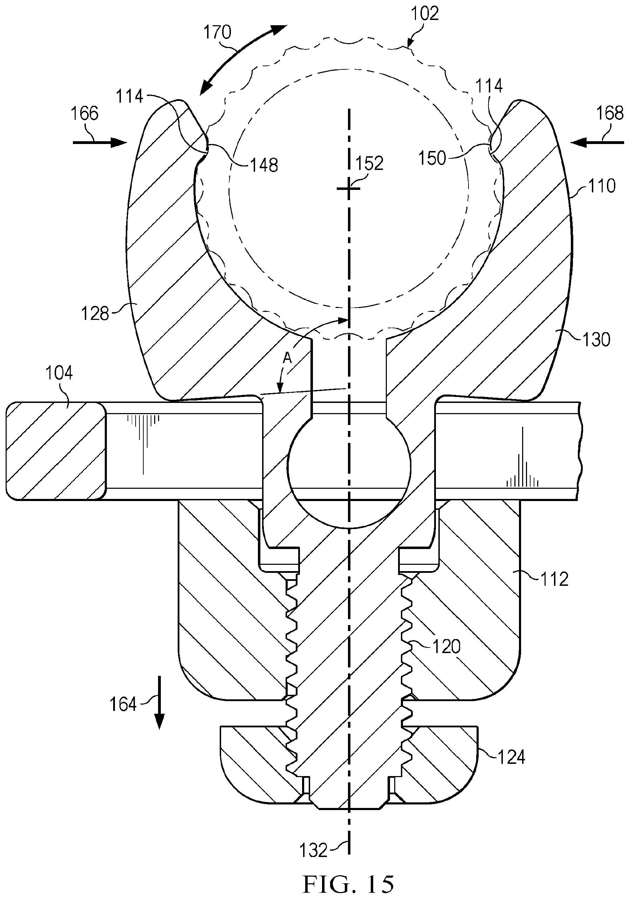

Continuing to refer to FIGS. 11-14, certain structural features are described with their respective functions and benefits. The first clip arm may include a first clip arm inner surface 142, and the second clip arm may include a second clip arm inner surface 144. The first clip and second clip inner surfaces 142, 144 can each define a locking tube facing surface having a constant radius of curvature R1, represented in FIG. 11 by the portion of the respective surfaces that follow a first imaginary circular line 146 centered about a center point 152 that corresponds to the location of the locking tube 102 central axis 116 during use. As can be understood, the diameter of the first imaginary circular line 146 substantially equals the maximum diameter D1 of the locking tube 102. The constant radius of curvature R1 of each tube clip facing surface can be disrupted by an inwardly extending protrusion. In an embodiment, the constant radius of curvature R1 of each tube clip facing surface can be disrupted by a plurality of inwardly extending protrusions. For example, in the example shown in FIG. 11, the constant radius of curvature R1 is disrupted near distal portions of each of the first clip arm 128 and the second clip arm 130 at an inwardly-oriented first protrusion 148 and an inwardly-oriented second protrusion 150, respectively, with "inwardly" being in the direction of center point 152 (e.g., radially inward). The presence of, the position of, and the general shape of the first protrusion 148 and the second protrusion 150 provide a benefit to the cluster derotation assembly 100 by being suitable for engaging one or more of the axially-aligned grooves 114 of the locking tube 102 during use, as depicted in FIG. 15. By engaging at least one of the axially-aligned grooves 114 of the locking tube 102, axial movement, that is, axial slippage between the tube clip 110 and the locking tube 102 (e.g., relative movement between the tube clip 110 and the locking tube 102) is minimized or eliminated. Minimizing or eliminating axial slippage between the tube clip and the locking tube about its central axis 116 aids in a more controlled spinal derotation by minimizing or eliminating undesired axial slippage being imparted to the pedicle screws to which the locking tubes are affixed.

The first protrusion 148 and the second protrusion 150 extend inwardly of the first imaginary circular line 146. The tips of the first protrusion and the second protrusion 150 extend inwardly from a proximal portion at the first clip arm inner surface 142 and the second clip arm inner surface 144, respectively, to a distal portion that lies tangential to a second imaginary circular line 154 centered about the center point 152 and having a radius of curvature R2, wherein R2 is less than R1. The diameter of the second imaginary circular line 154 can be approximately, or exactly, equal to the diameter D2, as shown in FIG. 5, such that the first protrusion 148 and/or the second protrusion 150 partially or fully engage an axially-oriented groove 114. The position of each of the first protrusion 148 and the second protrusion 150 ensure that at least one will engage an axially-aligned groove 114 of the locking tube 102. In an embodiment, the spacing of both of the first protrusion 148 and the second protrusion 150 ensure that both will engage a respective axially-aligned groove 114 of the locking tube 102.

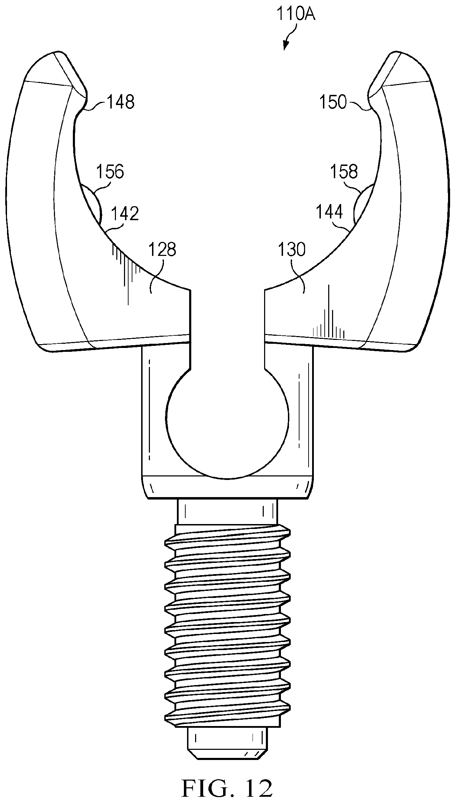

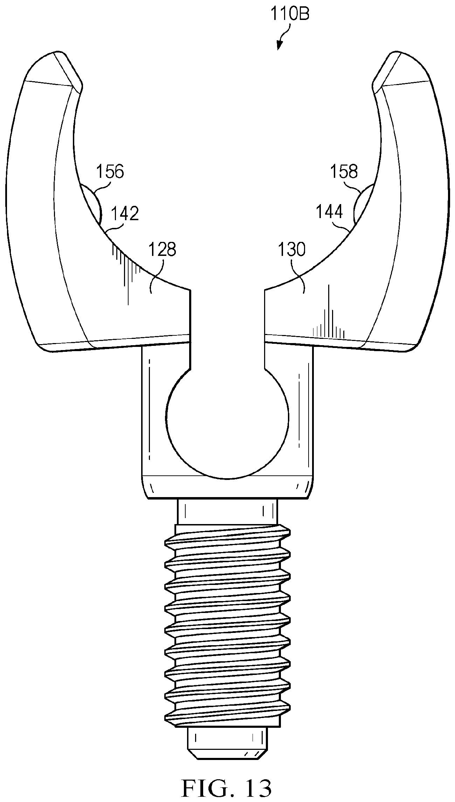

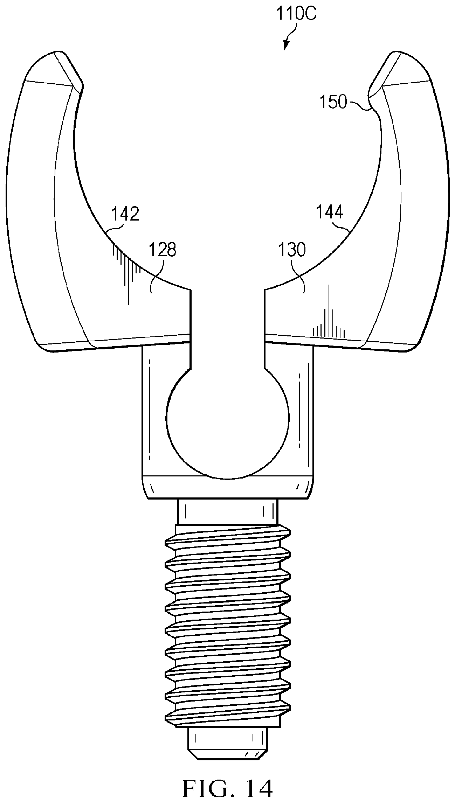

In general, therefore, a tube clip 110 of the present disclosure is intended to work in conjunction with a locking tube 102 having at least one axially-aligned groove 114. At least one tube clip protrusion, e.g., a first protrusion 148 can be positioned in use such that a distal end thereof protrudes at least partially into an axially-aligned groove 114 on the locking tube 102. The number and spacing of tube clip protrusions can be determined as desired. For example, as shown in FIG. 12, in an embodiment a tube clip 110A can have four protrusions, two protrusions 148 and 150 at or near a distal end of each tube clip arm, and two protrusions 156 and 158 that extend from the tube clip inner surfaces. As shown in FIG. 13, in an embodiment a tube clip 110B can have two protrusions 156, 158 that each extend from the tube clip inner surfaces, and the distal ends of the tube clip arms can be smooth, and protrusion-free. As depicted in FIG. 14, in an embodiment, a tube clip 110C can have only one protrusion, such as the protrusion 150 shown near the distal end of one of the tube clip arms such as, for example, the second clip arm 130.

Referring again to FIG. 11, in a method of use, a tube clip 110 can be urged orthogonally to a locking tube in the direction of arrow 164 until the locking tube is seated generally against the inner surfaces of the two tube clip arms. If necessary, one or both of the first clip arm 128 and the second clip arm 130 can flex outwardly away from the threaded shaft axis 132, permitting one or more protrusions, such as one or both of the first protrusion 148 and the second protrusion 150, to "ride" over the surface(s) of the locking tube until the locking tube is in position, such that its central axis 116 is co-axially aligned with the center point 152 and one or both of the first protrusion 148 and the second protrusion 150 can "snap" into one or more axially-aligned grooves of the locking tube. The tube clip 110 can be inserted through the link member 104 (e.g., slot 122), a wingnut 112 can be threaded on sufficiently to place a tensioning force on the tube clip 110 in the direction of arrow 164, thereby sufficiently securing the tube clip 110, and the locking tube, to the link member 104.

Further as can be understood with continuing reference to FIG. 11, in an embodiment, when the tube clip 110 is in a relaxed state (e.g., first and second clip arms are in a first position or normal state), that is prior to being tightly secured to the link member 104 by being tightened by the wing nut 112, the bearing surfaces of the shoulder members 126 each lie substantially in a plane that is not parallel to the surface of the link member 104 (represented in FIG. 11 by dashed lines). Rather, the bearing surfaces of the shoulder portions make an angle A relative to the threaded shaft axis 132, and, in use prior to being secured, with a surface of the link member 104. In the illustrated embodiment, the angle A is not 90 degrees. As shown in FIG. 11, the angle A is an angle of greater than 90 degrees.

As can be understood from the description herein, and with reference to FIG. 15, in use the structure of the tube clip 110 in any of the various embodiments disclosed facilitates a better connection with the locking tube 102. In an embodiment, the better connection includes a connection that minimizes or eliminates axial movement of the locking tube, that is, movement in the direction of arrow 170 when used in a cluster derotation assembly 100. As the wing nut 112 is threaded onto the threaded shaft 120 and engages the link member 104 a tensioning force in the direction of arrow 164 is applied to the tube clip 110. The tensioning force tends to draw the threaded shaft 120 of the tube clip 110 in the direction of arrow 164 such that the bearing surfaces of the shoulder members 126 of the tube clip 110 are drawn into interfacing contact with the surface of the link member 104. Coincidentally with the drawing down of the bearing surfaces, the distal ends of the first clip arm 128 and the second clip arm 130 are urged inwardly in the direction of the arrows 166 and 168, respectively, toward the threaded shaft axis 132, thus adding a tightening force on the locking tube 102, when in use (e.g., first and second clip arms are in a second position or secured state). Further, the protrusion(s) can be more forcefully and securely urged into axially-aligned groove(s) 114. For example, as shown in FIG. 15, a first protrusion 148 is urged into an axially aligned groove 114. Likewise, a second protrusion 150 is urged into another axially aligned groove 114.

Variations of a cluster derotation assembly 100 are contemplated. For example, the multiple locking tubes 102, linked by link members 104, can be replaced by a single handle member from which extend the functional equivalent of the plurality of linked locking tubes. However configured, the cluster derotation assembly 100 can facilitate simultaneous application of manipulative forces to multiple pedicle screws which are implanted in a like number of vertebra, without undesired axial slippage between a tube clip and a locking tube, and the corresponding undesired slippage being applied to the pedicle screws. This has the effect of permitting the gross, *en bloc* application of sufficient derotative forces to affected segments of the spinal column in a sufficiently dispersed manner, without undesired axial slippage of a tube clip on a locking tube, as to avoid injury to any one vertebra or isolated spinal column segment. This, in turn, can facilitate a successful entire-spine, 3D derotation of a scoliosis patient to near normal parameters.

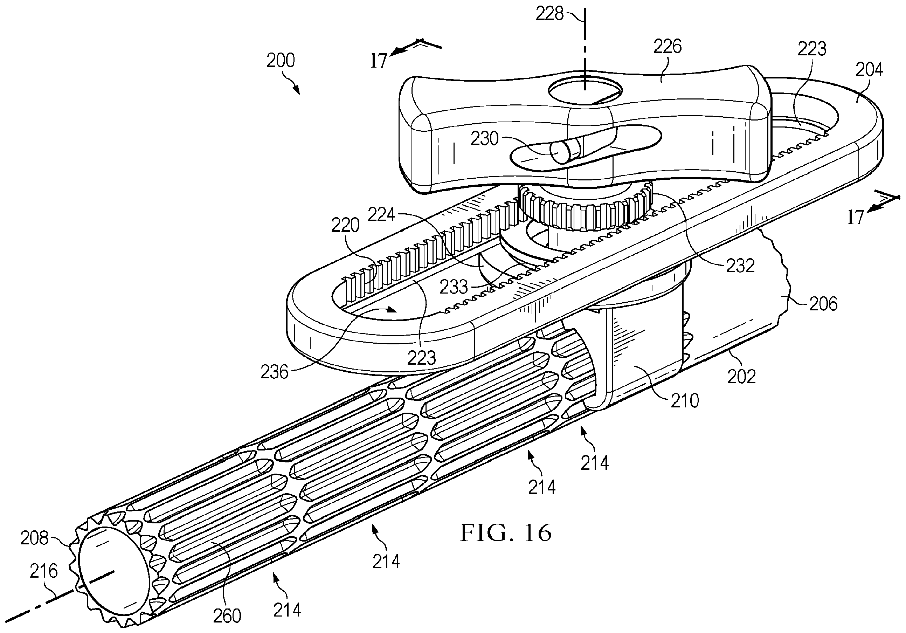

Referring now to FIGS. 16-19, there is shown an example system of a representative cluster derotation assembly 200. As depicted in FIG. 16, the cluster derotation assembly 200 is configured to join to a locking tube 202 and secures by a link member 204 that can securely join the locking tube 202 to another locking tube or tool (not shown) in a rigid configuration such that they together act in unison during use. As will be understood from the description herein, the cluster derotation assembly 200 facilitates a locking tube connection that prevents vertical, axial and lateral slippage between the derotation assembly and the locking tube.

Each locking tube 202 can have a distal end portion 206 and a proximal end portion 208 and can be configured to securely join to a bone anchor (not shown), as discussed above. The link member 204 can be joined at a location intermediate the distal end portion 206 and the proximal end portion 208. The locking tube 202 has a plurality of externally disposed bands 214 of axially-aligned grooves 260 (e.g., each band comprises a cluster of axially-aligned grooves). In the illustrated embodiment of FIG. 16, four bands 214 of axially-aligned grooves 260 are shown, the bands of axially-aligned grooves 260 being in a spaced relationship relative to one another along a longitudinal axis (e.g., a longitudinal axis coaxial with the central axis 216). The bands of axially-aligned grooves 260 function to provide a surface by which the axial rotation of the locking tube during use is minimized or eliminated. As will be appreciated from the disclosure below, the number, size, and placement of the grooves 260 is determined and implemented in conjunction with the size, shape, and design of a tube clip 210. In an embodiment there can be from about 12 to about 36 grooves 260. Each locking tube is generally cylindrically shaped and extends a suitable length about a central axis 216.

A tube clip 210 can extend through the link member 204 to the locking tube 202 in a similar fashion as described above. Each tube clip 210 can be secured by the action of a tightening member, such as a wing nut being threaded and tightened onto a portion of the tube clip 210 (as described above) or other securement member, including, for example, a cam locking mechanism (not shown). In the embodiment of the cluster derotation assembly 200 illustrated, however, additional elements contribute to the benefit of minimizing vertical, axial and lateral slippage during use.

The cluster derotation assembly 200 includes a link member 204 that is shown in the representative configuration of an elongated oval shape. The elongated oval shape can alternately be described as "race track" shaped, and can be rectangular. In general, the link member 204 can be any shape, but the interior open portion is bounded on the two long sides by parallel side members having internally oriented teeth, as described in more detail below. The link member 204 can have a length and width sufficient to join two or more locking tubes 202 during use. The link member 204 defines on its long side portions opposing rows of link teeth 220, which can be uniformly spaced, internally oriented projections, similar in nature to gear teeth. Likewise, the link member 204 has an internally projecting ridge 223 that extends from the two long side portions that can cooperate with a groove 233 of a tube clip mating member 224 in sliding engagement, as discussed in more detail below. A handle 226 can be operatively joined to the tube clip 210, for example at a tube clip shaft 221 defining a shaft axis 228, and configured to be rotated about shaft axis 228 to tighten the tube clip 210 on the locking tube 202 in a similar manner as discussed above, and to also engage a locking member 232 with the link member 204. The locking member 232 can be a generally circular shaped toothed member having externally oriented, uniformly spaced locking teeth 234 which can be, as described below, urged into locking engagement with the link teeth 220 of the link member 204. The handle 226 can be joined to the shaft 221 of the tube clip 210 in a pinned arrangement by pin 230, which according to the configuration of a slot 246 in the handle 226 can permit a partial turn of the handle 226. For example, in an embodiment, the handle and tube clip connection is configured to tighten the tube clip with a quarter turn of the handle 226.

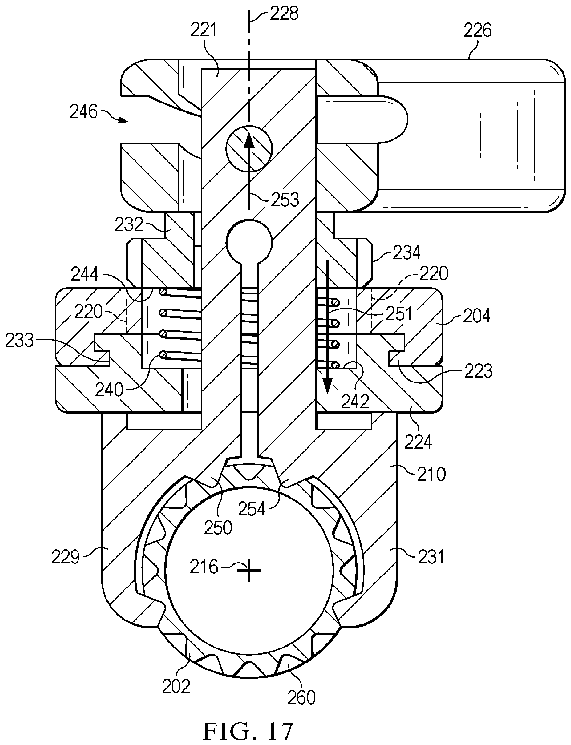

Referring now to FIG. 17 in conjunction with FIG. 16, the components, configuration, and operation of the cluster derotation assembly 200 are further described. In the configuration illustrated in FIGS. 16 and 17 the link member 204 is free to move relative to the other components, constrained only by the internally projecting ridge 223 of the link member 204 which matingly cooperates with the groove 233 of the tube clip mating member 224 in sliding engagement. A spring member 240, such as a coil spring, provides a biasing force between a first bearing surface 242 of the tube clip mating member 224 and a second bearing surface 244 of the locking member 232, such that the locking member 232 is urged out of an open area 236, such that the locking teeth 234 of the locking member 232 are not engaged with the link teeth 220 of the link member 204. In this configuration, as shown, the internally projecting ridge 223 of the link member 204 engages the groove 233 of the tube clip mating member 224, thereby providing the structure to operatively join all the components in a relationship that keeps all but the link member 204 in an arrangement defined by an alignment with the shaft axis 228. The link member is slideably joined to be moveable in a direction perpendicular to the shaft axis 228.

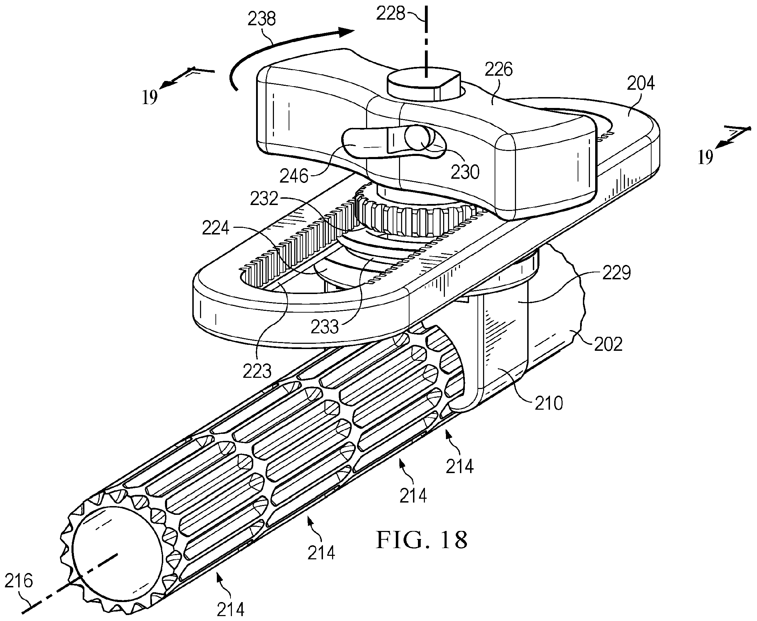

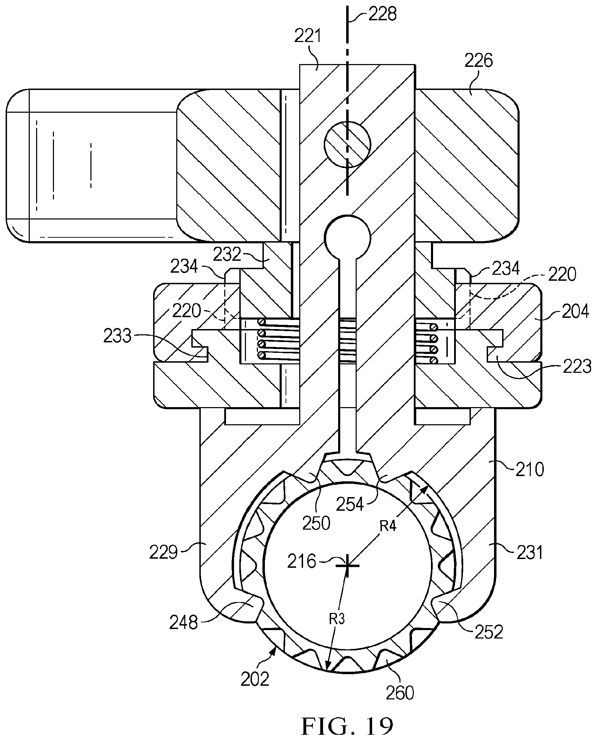

Referring now to FIGS. 18 and 19, the cluster rotation tool 200 is shown in its locked, in-use configuration, achieved by turning the handle 226, such as in the direction of arrow 238 to pivot about axis 228. Handle 226 has defined therein a sloped slot 246 in which the pin 230 resides and which upon turning, due to its sloped nature forces the handle, via engagement with the pin 230 that engages a portion of the tube clip 210 in a fixed relationship, to urge the portion of tube clip 210 gripping the locking tube to draw closer to the handle, that is, to be drawn in the direction of the arrow 253 in FIG. 17. The turn of the handle has the corresponding effect of applying a force on the locking member 232, urging it in the relative direction of arrow 251 in FIG. 17, thus urging the locking member 232 against the biasing force of the spring member 240 and into the open area 236 of the link member 204. The locking member 232 is sized to have a diameter such that the locking teeth 234 engage in an alternating, intermeshing manner, with the link teeth 220 of the link member 204, as depicted more clearly in FIG. 19. As can be understood, when the locking teeth 234 of the locking member 232 are engaged with the link teeth 220 of the link member 204, the link member 204 is prevented from moving in any direction perpendicular to the axis 228. Further, the turn of the handle draws the tube clip 210 against the tube clip mating member 224 and causes it to grip the locking tube 202 to constrain axial rotation of the locking tube 202 about axis 216 during use.

As depicted in FIGS. 17 and 19, the grooves 260 of the band 214 of axially-aligned grooves 260 extend inwardly from the outside diameter R3 of the locking tube 202. In use a portion of a tube clip 210 will be operationally adjacent the locking tube 202 at the outside diameter, while one or more portions of the tube clip 210 can be operationally disposed in one or more grooves 260. Referring to FIG. 19, the tube clip 210 can have at a distal portion thereof a portion similar in structure and function to the tubes clip disclosed above and which is designed to grip the locking tube 202. In an embodiment, a first clip arm 229 and a second clip arm 231 have locking tube-facing relatively smooth surfaces that define a generally constant radius of curvature R4. In an embodiment, radius of curvature R3 and radius of curvature R4 are substantially equal. The inwardly oriented, locking tube-facing surface of each of the clip arms can be disrupted by an inwardly extending protrusion. In an embodiment, the constant radius of curvature R4 of each tube clip facing surfaces can be disrupted by a plurality of inwardly extending protrusions. For example, in the example shown in FIG. 19, the surface having constant radius of curvature R4 is disrupted by one or more inwardly-oriented first protrusions, with "inwardly" being in the direction of the center of the locking tube 202, e.g., generally toward axis central 216. In an embodiment, the first clip arm 229 can have a first inwardly-oriented protrusion 248 disposed near a distal portion of the first clip arm 229, and a second inwardly-oriented protrusion 250 at the proximal portion of the first clip arm. Likewise, the second clip arm 231 can have a third inwardly-oriented protrusion 252 disposed near a distal end of the second clip arm 231, and a fourth inwardly-oriented protrusion 254 at a proximal portion of the second clip arm. The presence of, the position of, and the general shape of the protrusions provide a benefit to the cluster derotation assembly 200 by being suitable for engaging one or more of the axially-aligned grooves 260 of the locking tube 202 during use, as depicted in FIG. 19. By engaging at least one of the axially-aligned grooves 260 of the locking tube 202, axial movement, that is, axial slippage (e.g., relative movement between the locking tube and the tube clip) between the tube clip 210 and the locking tube 202 about axis 216 is minimized or eliminated. Minimizing or eliminating axial slippage between the tube clip and the locking tube aids in a more controlled spinal derotation by minimizing or eliminating undesired axial slippage being imparted to the pedicle screws to which the locking tubes are affixed.

The link member 204 can be a relatively rigid with a size and shape sufficient to provide the benefit of being a relatively rigid connector as disclosed herein. In some embodiments, the link member 204 may also be lengthened to allow connection to more locking tubes (e.g., connection to 3, 4, 5, 6, 7 locking tubes, etc.). The link member 204 can be a rigid body having defined therein an open slot 236 through which a shaft 2 of the tube clip traverses during use. The width dimension of the open slot 236 and the diameter of the locking member can be predetermined to ensure engagement of the teeth with the locking teeth of the locking member.

In general, therefore, a tube clip 210 of the present disclosure is intended to work in conjunction with a locking tube 202 having at least one axially-aligned groove 260. At least one tube clip protrusion can be positioned in use such that it protrudes at least partially into an axially-aligned groove 260 on the locking tube 202. The number and spacing of grooves and protrusions can be determined as desired.

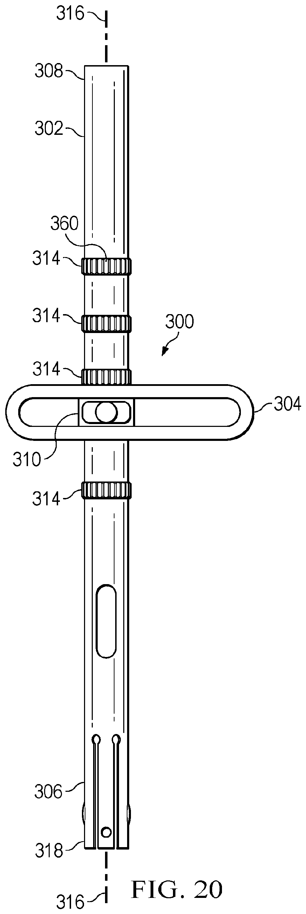

Referring now to FIGS. 20-22, there is shown an example system of a representative cluster derotation assembly 300. As depicted in FIG. 20, the cluster derotation assembly 300 is configured to join to locking tube 302, by a link member 304 that secures the locking tube 302 to another locking tube or tool (not shown) in a rigid configuration such that they together act in unison during use (e.g., locking tube 302 is fixed, for example, axially fixed, relative to each additional tool). Each locking tube has a distal end portion 306 being configured to securely join to a bone anchor, and a proximal end portion 308. The link member 304 can be joined at a location intermediate the distal end portion 306 and the proximal end portion 308. A tube clip 310 can extend through the link member 304 to the locking tube 302 in a manner as described above. Each tube clip 310 can be secured by the action of a wing nut being threaded and tightened onto a portion of the tube clip 310 (as described above) or other securement member, including, for example, a cam locking mechanism (not shown). Portions of the locking tube 302 are characterized by externally disposed bands of axially-aligned ridges 314 defining grooves 360. The number, size, and placement of the grooves 360 can be varied depending on design considerations. As will be appreciated from the disclosure below, the number, size, and placement of the grooves 360 is determined and implemented in conjunction with the size, shape, and design of the tube clip 310. In an embodiment there can be from about 12 to about 36 grooves 360.

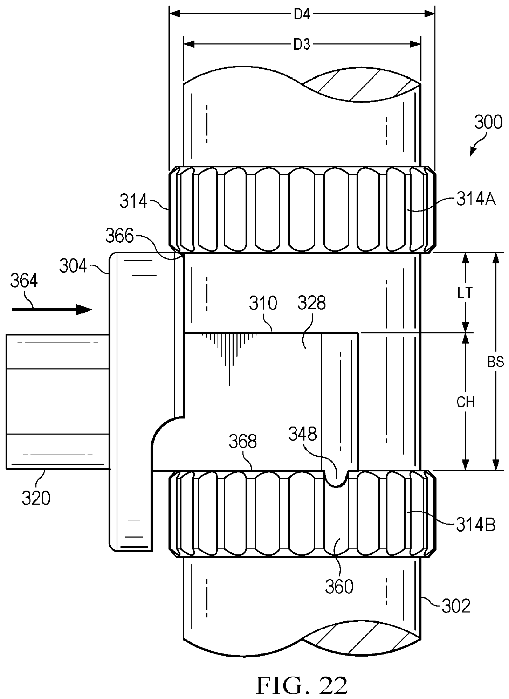

As depicted in FIG. 22, the tops (e.g., peaks) of the ridges of the band of axially-aligned ridges 314 extends externally outwardly from the outside diameter D3 of the locking tube 302 to define a maximum diameter D4 of the bands of axially-aligned ridges 314 (denoted as axially-aligned ridges 314A and 314B for further description below). In use a portion of a tube clip 310 will be operationally adjacent the locking tube at the outside diameter D3, while one or more portions of the tube clip 310 can be operationally disposed in one or more grooves 360.

The link member 304 can be a relatively rigid with a size and shape sufficient to provide the benefit of being a relatively rigid connector as disclosed herein. In some embodiments, the link member 304 may also be lengthened to allow connection to more locking tubes (e.g., connection to 3, 4, 5, 6, 7 locking tubes, etc.). The link member 304 can be a rigid body having defined therein an open slot 322 through which a shaft 320 of the tube clip traverses during use.

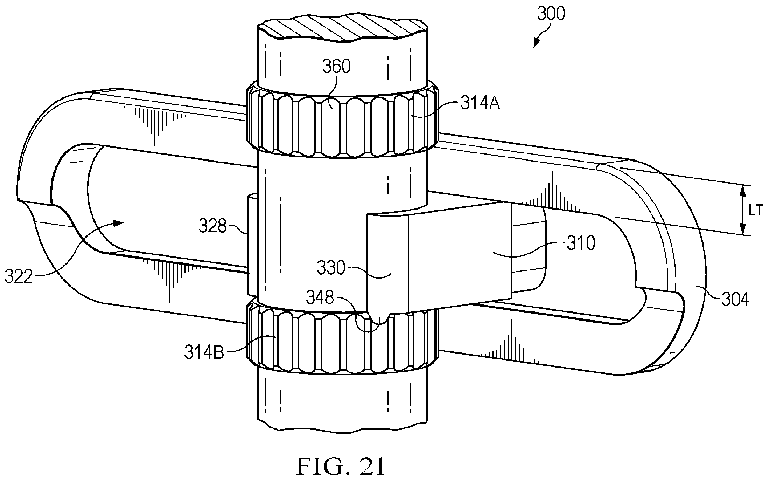

FIG. 21 is perspective view of a portion of the cluster derotation assembly 300 shown in FIG. 20, and FIG. 22 is a side view of a portion of the cluster derotation assembly 300 shown in FIG. 20. The tube clip 310 has a first clip arm 328 and the second clip arm 330 that are joined to shaft 320 in a cantilevered configurations, such that each of the first clip arm 328 and the second clip arm 330 can be urged into a seating position around the outside diameter D3 of locking tube 302.

Continuing to refer to FIGS. 21 and 22, certain structural features are described with their respective functions and benefits, which except for the differences noted herein can be the same as or similar to the corresponding features disclosed above for cluster derotation assembly 100. One or both of the first clip arm 328 and the second clip arm 330 can have an axially-oriented pin that is sized, shaped, and positioned to fit, e.g., "lock," into a portion of a groove 360. In the illustrated embodiment, each of the first clip arm 328 and the second clip arm 330 has a pin 348 disposed at their respective distal end portions. As shown, pin 348 extends from the distal end portion in a direction axially aligned with the axis 316, rather than radially inwardly as the protrusions did on tube clips 110 described above. Pins 348 can be secured and remain secured in operation when certain dimensional parameters of the components of cluster derotation assembly 300 are satisfied, as depicted in FIGS. 21 and 22. The link member can have a portion having a link thickness dimension LT, for example on an upper length portion, as indicated in FIG. 21. The clip arm, for example first clip arm 328 shown in FIG. 22 can have a clip height dimension CH measured in a direction corresponding to axis 316. The bands of axially-aligned ridges 314 can be separated by a band separation distance BS. In an embodiment, the link thickness dimension LT and the clip height dimension CH add to substantially equal the band separation distance BS. When the tube clip 310 is urged onto the locking tube 302 and positioned such that a pin 348 is engaged in a groove 360, the link member 304 can be urged toward the locking tube in the direction of arrow 364. In the contacting and operating position, the top surface of the linking member presses against the bottom surface of a top band of axially-aligned ridges 314A, essentially forming a bearing surface at the upper interface 366. Likewise, the lower surface of the tube clip 310 presses against an upper surface of a lower band of axially-aligned ridges 314B, essentially forming a bearing surface at the lower interface 368. In this manner constraining forces due to the fit of the link member 304 against the tube clip 310 and in contact with the locking tube 302 constrains the pin 348 to remain disposed in groove 360, thereby further constraining any axial rotation of the locking tube during use of the cluster derotation assembly 300. That is, in a similar manner as described above with respect to cluster derotation assembly 100, and cluster derotation assembly 200, in cluster derotation assembly 300 by engaging at least one pin 348 in at least one of the grooves 360 of the locking tube 302, axial movement, that is, axial slippage between the tube clip 310 and the locking tube 302 is minimized or eliminated.

In general, therefore, in certain embodiments a tube clip of a cluster derotation assembly is intended to work in conjunction with a locking tube having at least one axially-aligned groove. At least one tube clip protrusion can be positioned in use such that it protrudes at least partially into an axially-aligned groove on the locking tube.

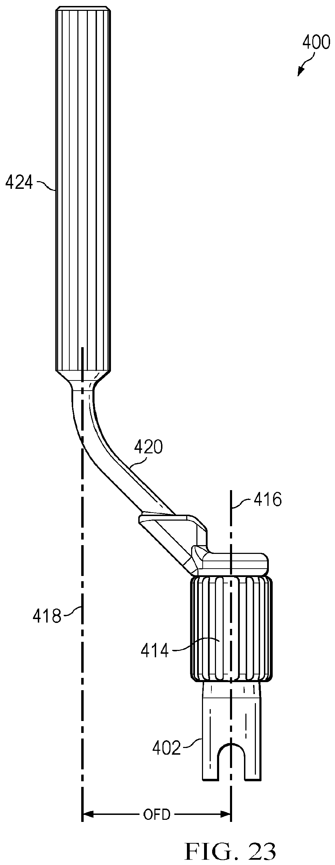

Referring now to FIG. 23, one or more of the locking tubes 402 can be an offset locking tube 400. A cluster derotation assembly as disclosed herein can utilize one or more offset locking tubes 400. The offset locking tube 400 can have a locking tube member 402 that can be in every respect identical to locking tubes described herein, including having a tube central axis 416 and axially-aligned grooves 414 to which can be joined a tube clip. Integral with, or joined to, the locking tube member 402 is a locking tube handle 420. The locking tube handle 420 can have a handle portion 424. The handle portion 424 can have a handle central axis 418 that can be substantially parallel to, and offset from the tube central axis 416 an offset distance OFD. The handle central axis 418 offset from the tube central axis 416 a sufficient amount to suitably allow for certain manipulative techniques to be successfully achieved. For example, a cluster derotation assembly 100 utilizing an offset locking tube 400 can be utilized to facilitate manipulating hyperlordotic spines, as the tops of the devices at cojoined levels will not interfere with each other.

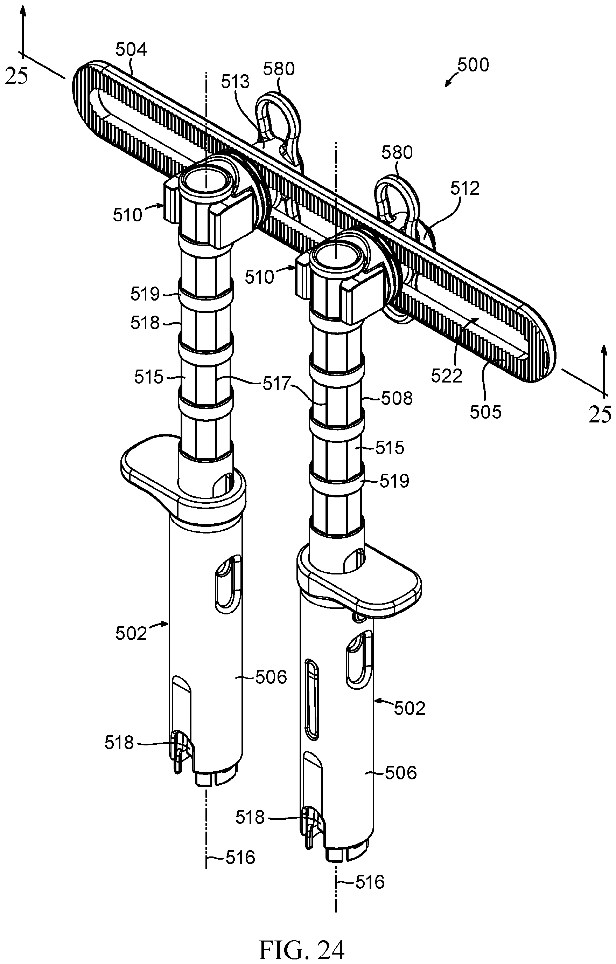

Referring now to FIG. 24, there is shown another example system of a representative cluster derotation assembly 500 configured to join to a plurality of locking tubes 502, by a link member 504 that secures each of the plurality of locking tubes 502 in a rigid configuration such that they together act in unison during use. Similar to those depicted in FIG. 2, each locking tube 502 has a distal end portion 506 being configured to securely join to a bone anchor, and a proximal end portion 508, to which can be joined the link member 504. A plurality of tube clips 510, at least one for each locking tube 502, can extend through the link member 504 to the locking tube 502, at or near the proximal end portion 508. Each tube clip 510 is secured by the action of a nut or similar connector (e.g., wing nut 512) being threaded and tightened onto a portion of the tube clip 510. As described in more detail below, a portion of the locking tube 502 may include a plurality of externally disposed, axially-aligned faces 515, the intersections of which define a plurality of axially-aligned points 517. The points 517 may include but are not limited to points, edges, and/or corners. The plurality of axially-aligned faces 515 and points 517 can be separated by diametrical rings 519 along a length of the proximal end portion 508. In certain embodiments, the plurality of axially-aligned faces 515 and a plurality of axially-aligned points 517 can be arranged in one or more sections about a circumference of the locking tube 502, and the diametrical rings 519 can provide axial boundaries for such sections. The axially-aligned faces 515 and points 517 function to provide a surface or surfaces by which the axial rotation of the locking tube 502 relative to the link member 504 and the one or more tube clips 510 during use is minimized or eliminated. The diametrical rings 519 function to minimize or eliminate axial translation of the locking tube 502 within the tube clips 510. Similar to those depicted in FIG. 2, each locking tube 502 is generally cylindrically shaped (e.g., and/or generally tubular shaped) and extends a suitable length about a central axis 516 (e.g., longitudinal axis). The locking tube 502 includes a distal end portion 506 containing a collet 518 configured to slide over and reversibly engage the head segment of a pedicle screw as described herein.

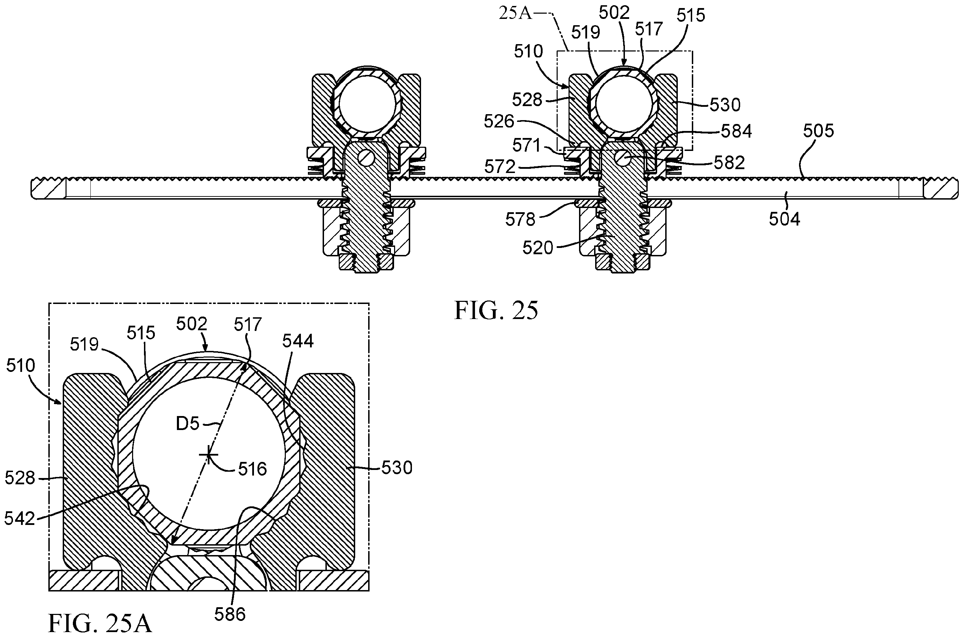

As discussed above, each locking tube 502 has a plurality of externally disposed, axially-aligned faces 515 and points 517, the number, size, and placement of which can be varied depending on other design considerations. As described with respect to embodiments described herein, the number, size, and placement of the axially-aligned faces 515 and points 517 are determined and implemented in conjunction with the size, shape, and design of the tube clip 510. In an embodiment, there can be from about 4 to about 24 of each of the axially-aligned faces 515 and points 517 about a circumference of the locking tube 502. In the illustrated embodiment of FIGS. 24, 25, and 25A, there are 8 axially-aligned faces 515 and 8 points 517. As depicted in FIGS. 25 and 25A, the axially-aligned points 517 define a maximum diameter D5 of the portion of the locking tube 502 having axially-aligned faces 515. As will be described more fully below, in operation, a portion of a tube clip 510 will be operationally adjacent the locking tube 502 at the maximum diameter D5, while one or more portions of the tube clip 510 can be operationally disposed operationally adjacent portions of the locking tube 502 at a radial distance less than the maximum diameter D5.

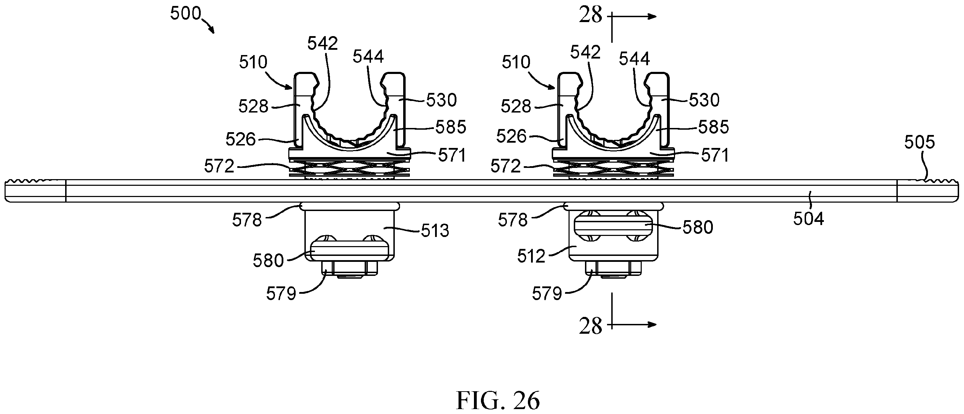

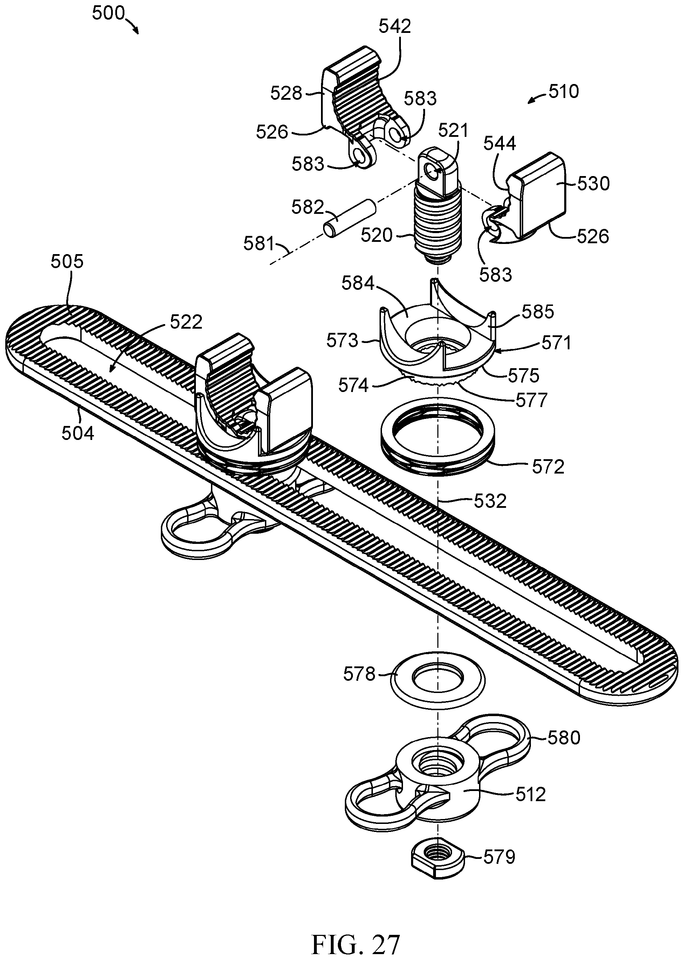

Referring now to FIG. 26, there is depicted an embodiment of a cluster derotation assembly 500 configured to join to two locking tubes such as, for example, locking tube 502. It will be appreciated, however, that more than two locking tubes 502 can be joined by the cluster derotation assembly 500. As an example, two tube clips 510 are shown mounted on the link member 504, and each is secured in place by a nut or similar connector (e.g., wing nuts 512, 513) connected to (e.g., threaded on to) a shaft of the tube clip 510 (e.g., a threaded shaft 520 of the tube clip 510). Link member 504 is a rigid body having defined therein an open slot 522 through which the shaft (e.g., threaded shaft 520) of the tube clip 510 traverses. As shown in FIG. 27, link member 504 has a textured top surface 505 that can engage with a corresponding textured surface of a knurled washer, as will be described in greater detail below.

As depicted in FIGS. 24-28, the threaded shaft 520 is sized to have a diameter less than the width of the open slot 522 of the link member 504, such that, in use, the threaded shaft 520 extends through the link member 504. Referring to FIG. 27, the threaded shaft 520 can extend through, on a first side of the link member 504, a knurled washer 571 and a wave spring 572. The knurled washer 571 can have an upper portion 573 and a lower portion 574, where the diameter of the upper portion 573 is larger than the diameter of the lower portion 574 and the diameter of the lower portion 574 is larger than the width of the open slot 522 of the link member 504 but is sized to extend through an opening 576 defined by the wave spring 572. Accordingly, and as shown in FIG. 27, an inner diameter of the wave spring 572 can also be larger than the width of the open slot 522. In operation, a lower surface 575 of the upper portion 573 can engage an upper surface of the wave spring 572. As mentioned above and described in greater detail below, the lower portion 574 of the knurled washer 571 can have a textured bottom surface 577 that can engage with the textured top surface 505 of the link member 504.

The threaded shaft 520 can extend through, on a second side of the link member 504, a derotation washer 578, a wing nut (e.g., 512), and a weld washer 579. The derotation washer 578 can be positioned between the link member 504 and the wing nut (e.g., 512), such that an outer diameter of the derotation washer 574 can be larger than the width of the open slot 522 of the link member 504. The wing nut (e.g., 512) threads onto the threaded shaft 520, and weld washer 579 can be secured to the end of the threaded shaft 520 to prevent the wing nut (e.g., 512) from threading off the end of the threaded shaft 520. Each of the wing nuts (e.g., 512, 513) can include a pair of handles 580, and as shown in FIGS. 26 and 27, the respective pairs of handles 580 for wing nuts 512, 513 can nest with each other. For example, the pair of handles 580 on the first wing nut 512 can be positioned closer to a first end of the first wing nut 512 (e.g., closer to the link member 504), while the pair of handles 580 on a second wing nut 513 can be positioned closer to a second end of the second wing nut 513 (e.g., further from the link member 504). When in a secured position, the respective pairs of handles 580 of the first and second wing nuts 512, 513 can avoid interference with each other, even when the first and second wing nuts 512, 513 are positioned close enough to each other such that rotational circumferences of the respective pairs of handles 180 would be overlapping if located in the same horizontal plane.

Referring again to FIG. 27, a top portion of the threaded shaft 520 can define a shaft pinhole 521. The shaft pinhole 521 defines a pivoting axis 581 and can receive a pin 582. In an embodiment, the pivoting axis 581 can be perpendicular or substantially perpendicular to the threaded shaft axis 532. Lower portions of each of a first clip arm 528 and a second clip arm 530 can define complementary clip arm pinholes 583, each of which can be aligned with the shaft pinhole 521 and can likewise receive the pin 582. Each of the first clip arm 528 and the second clip arm 530 can be rotatably coupled to the threaded shaft 520, connected to the shaft 520 by the pin 582, such that each of the first clip arm 528 and the second clip arm 530 can extend from the threaded shaft 520 and be rotatable about the pivoting axis 581. When the wing nut (e.g., 512) is tightened onto the threaded shaft 520, an upper surface 584 of the knurled washer 571, urged by the collective force of the derotation washer 578, link member 504, and wave spring 572, can engage respective shoulder members 526 of the first clip arm 528 and the second clip arm 530, such that each of the first clip arm 528 and the second clip arm 530 can be urged to pivot inwardly toward the threaded shaft axis 532 to, for example, further secure a locking tube 502. When the wing nut (e.g., 512) is loosened, each of the first clip arm 528 and the second clip arm 530 can be allowed to pivot outwardly from the threaded shaft axis 532. In an embodiment, the upper portion 573 of the knurled washer 571 can further include four projections 585 between which the first clip arm 528 and the second clip arm 530 can be received.

The first clip arm 528 and the second clip arm 530 may include a first clip arm inner surface 542 and a second clip arm inner surface 544, respectively. The first and second clip inner surfaces 542, 544 can each define a locking tube facing surface having a plurality of axially-aligned ridges 586 and, apart from peaks and valleys of the plurality of axially-aligned ridges 586, a constant radius of curvature, as described above in other embodiments. The plurality of axially-aligned ridges 586 can be sized to complement the plurality of axially-aligned faces 515 and points 517. In use, one or more of the plurality of axially-aligned ridges 586 can engage one or more of the plurality of axially-aligned faces 515 and points 517, as shown in FIG. 25. In an embodiment, an angle formed at one or more of the plurality of axially-aligned points 517, by two of the plurality of axially-aligned faces 515, can be the same as that formed within one or more of the plurality of axially-aligned ridges 586, such that one or more of the plurality of axially-aligned ridges 586 can be sized to receive one or more of the axially-aligned points 517 and at least a portion of one or more of the axially-aligned faces 515. As shown in FIGS. 25 and 25A, the number of the axially-aligned ridges 586 may exceed the number of each of the axially-aligned faces 515 and points 517 to, for example, provide a multitude of gripping positions. It will be appreciated, however, that in other embodiments, a number of axially-aligned ridges can be the same as that for each of axially-aligned faces 515 and points 517 and/or axially-aligned ridges can have a reciprocal surface to that of a surface defined by axially-aligned faces 515 and points 517. By the engagement of at least one of the plurality of axially-aligned points 517 of the locking tube 502 with one or more of the plurality of axially-aligned ridges 586, rotational movement, or slippage, between the tube clip 510 and the locking tube 502 can be minimized or eliminated.

The plurality of axially-aligned faces 515 and points 517 can be separated by diametrical rings 519 along a length of the proximal end portion 508, as described above. In certain embodiments, spacing between each of the diametrical rings 519 (e.g., a length of a section) can be the same as or substantially the same as a width of the tube clip 510. The diametrical rings 519 can have a diameter that is greater than or equal to the maximum diameter D5 of the portion of the locking tube 502 having axially-aligned faces 515, such that the diametrical rings 519 can minimize or eliminate axial translation of the locking tube 502 within the tube clips 510 (e.g., the diametrical rings may minimize or prevent one or more tube clip(s) 510 from translating along the longitudinal axis of a locking tube 502). Minimizing or eliminating rotational and axial slippage between the tube clip 510 and the locking tube 502 about or along its central axis 516 aids in a more controlled spinal derotation by minimizing or eliminating undesired rotational or axial slippage being imparted to the pedicle screws to which the locking tubes 502 are affixed.

In a method of use, a tube clip 510, in a relaxed state, can be urged orthogonally to a locking tube 502 until the locking tube 502 can be seated generally against the inner surfaces 542, 544 of the two tube clip arms 528, 530. As the locking tube 502 engages the tube clip 510 and moves toward the inner surfaces 542, 544, one or both of the first and second clip arms 528, 530 can flex outwardly away from the threaded shaft axis 532. As one or both of the first clip arm 528 and the second clip arms 530 flex outwardly, rotating about the pivoting axis 581, the shoulder members 526 of the first clip arm 528 and the second clip arm 530 can engage and exert a force upon the upper surface 584 of the knurled washer 571, which can cause compression of the wave spring 572. The wave spring 572 can be compressed enough to allow for the first clip arm 528 and the second clip arm 530 to flex outwardly, but it will be appreciated that once compression forces are removed, a wave spring can urge a knurled washer and first and second clip arms back to the relaxed state. In certain embodiments, the wave spring 572 can cause the knurled washer 571 to be biased toward the shoulders 526 of the first clip arm 528 and the second clip arm 530. In certain embodiments, the locking tube 502 can "snap" into position, such that one or more of the axially-aligned points 517 is received in one or more of the plurality of axially-aligned ridges 586. In certain embodiments, the central axis 516 of the locking tube 502 can be co-axially aligned with a center point between the inner surfaces 542, 544, when the locking tube 502 is secured within the tube clip 510. Once the locking tube 502 is so secured, the wing nut (e.g., 512) can be sufficiently threaded on shaft 520 to place a tensioning force on the tube clip 510, thereby placing the first and second clip arms 528, 530 in a secured state and sufficiently securing the tube clip 510, and the locking tube 502, to the link member 504.

As discussed above, the top surface 505 of the link member 504 can include grooves, which, in use, can engage with machined grooves on the bottom surface 577 of the lower portion 574 of the knurled washer 571. As shown in FIG. 27, the grooves of the top surface 505 can be parallel or substantially parallel to a length of the link member 504. The grooves on the bottom surface 577 of the lower portion 574 of the knurled washer 571 are machined to lock into the grooves on the top surface 505 of the link member 504, when the wing nut (e.g., 512) is sufficiently tightened, regardless of the angular positioning of the knurled washer 571 relative to the link member 504, such that when the first clip arm 528 and the second clip arm 530 are in a secured state and the tube clip 510 is sufficiently secured to the link member 504, the interaction between the grooves on the bottom surface 577 of the lower portion 574 of the knurled washer 571 and the grooves on the top surface 505 of the link member 504 can reduce or eliminate both rotation and translation of tube clip 510 relative to the link member 504.

The wave spring 572 can have a flat or substantially flat bottom surface, such that when the wing nut (e.g., 512) is loosened and the tube clip 510 is in a relaxed state, the wave spring 572 can easily slide over the grooves on the top surface 505 of the link member 504. Thus, the tube clip 510, in the relaxed state, can be effectively translated along a length of the link member 504 and/or rotated relative to the link member 504 to allow for greater degrees of freedom for assembling a surgical construct. Further, by minimizing or eliminating rotational and axial slippage between the tube clip 510 and the locking tube 502 and minimizing or eliminating both rotation and translation of tube clip 510 relative to the link member 504, such degrees of freedom can be effectively removed with the securement of the above-described components, such that equal loading can be distributed across all pedicle screws within the assembled surgical construct.

Thus, the apparatus, system, and method of the present disclosure facilitates application of forces to multiple spinal column segments, to achieve an over-all spinal column correction.

In an embodiment, a spinal derotation assembly kit may include one or more link members as described herein and two or more tube clips as described herein. In another embodiment, a spinal derotation assembly kit may include one or more locking tubes as described herein; one or more link members as described herein; and one or more tube clips as described herein. In some embodiments the kits herein may additionally include one or more offset locking tubes as described herein. In another embodiment, a spinal derotation assembly kit may include one or more offset locking tubes as described above herein; one or more link members as described herein; and one or more tube clips as described herein.

In an embodiment, a cluster derotation assembly derotation assembly includes a link member having a surface and an open slot through the link member; a first tube clip, the first tube clip including a shaft having external threads, a first clip arm extending from a first end of the shaft, and a second clip arm extending from the first end of the shaft, wherein the first and second clip arms are opposed to each other, and wherein the shaft is inserted through the open slot of the link member such that the first and second clip arms of the first tube clip are positioned on a first side of the link member and a second end of the shaft is positioned on a second side of the link member; a second tube clip, the second tube clip including a shaft having external threads, a first clip arm extending from a first end of the shaft, and a second clip arm extending from the first end of the shaft, wherein the first and second clip arms are opposed to each other, and wherein the shaft is inserted through the open slot of the link member such that the first and second clip arms of the second tube clip are positioned on a first side of the link member and a second end of the shaft is positioned on a second side of the link member; a first nut with an aperture disposed therein, the aperture including an internal thread that is threadingly engaged to the external thread of the shaft of the first tube clip, wherein when the first nut is tightened onto the threaded shaft of the first tube clip, the contact of the first tube clip with the surface of the link member causes the first and second clip arms to move inward toward a threaded shaft axis; a second nut with an aperture disposed therein, the aperture including an internal thread that is threadingly engaged to the external thread of the shaft of the second tube clip, wherein when the second nut is tightened onto the threaded shaft of the second tube clip, the contact of the second tube clip with the surface of the link member cause the first and second clip arms to move inward toward a threaded shaft axis axis.

This embodiment may further include wherein one or both of the first clip arm and the second clip arm of one or both the first tube clip and the second tube clip includes an inner surface configured to engage with a complementary surface of a locking tube. This embodiment may further include wherein the inner surface includes a plurality of axially-aligned ridges and the complementary surface of the locking tube includes a plurality of axially-aligned faces, wherein intersections of the plurality of axially-aligned faces define a plurality of axially-aligned points.

Further, this embodiment may also include wherein one or both of the first clip arm and the second clip arm of one or both the first tube clip and the second tube clip are rotatably coupled to the shaft. This embodiment may also include wherein one or both of the first tube clip and the second tube clip includes a knurled washer, wherein an upper surface of an upper portion of the knurled washer is configured to engage the first clip arm and the second clip arm. This embodiment may also include wherein one or both of the first tube clip and the second tube clip includes a wave spring, wherein the wave spring is configured to engage a lower surface of the upper portion of the knurled washer and an upper surface of the link member. This embodiment may also include wherein each of the upper surface of the link member and a bottom surface of a lower portion of the knurled washer has a machine grooved surface; and wherein the lower portion of the knurled washer extends through an opening defined by the wave spring, such that, when the respective nut is tightened onto the threaded shaft of the respective tube clip, the bottom surface of the lower portion of the knurled washer engages with the upper surface of the link member to reduce or eliminate rotation or translation of the respective tube clip relative to the link member. This embodiment may also include wherein a bottom surface of the wave spring is flat or substantially flat.

In addition, the embodiment may include wherein one or both of the first nut and second nut include wing nuts. Additionally, the embodiment may include wherein the first nut includes a first wing nut and the second nut includes a second wing nut, wherein the first and second wing nuts nest with each other.

In another embodiment, a cluster derotation assembly including a first locking tube having a distal end portion configured to securely join to a bone anchor and a proximal end portion having an outer surface; a second locking tube having a distal end portion configured to securely join to a bone anchor and a proximal end portion having an outer surface; a link member having a surface and an open slot through the link member; a first tube clip having a shaft extending through the open slot, a first clip arm extending from an end of the shaft, and a second clip arm extending from the end of the shaft, wherein one or both of the first clip arm and the second clip arm have an inner surface that is complementary to the outer surface of the proximal end portion of the first locking tube, wherein the first and second clip arms are opposed to each other, and wherein the first and second clip arms are movable between a first position away from a shaft axis and a second position toward a shaft axis such that the inner surface of one or both of the first clip arm and the second clip arm engages the outer surface of the proximal end portion of the first locking tube; a second tube clip having a shaft extending through the open slot, a first clip arm extending from an end of the shaft, and a second clip arm extending from the end of the shaft, wherein one or both of the first clip arm and the second clip arm have an inner surface that is complementary to the outer surface of the proximal end portion of the second locking tube, wherein the first and second clip arms are opposed to each other, and wherein the first and second clip arms are movable between a first position away from a shaft axis and a second position toward a shaft axis such that the inner surface of one or both of the first clip arm and the second clip arm engages the outer surface of the proximal end portion of the second locking tube.

Further, this embodiment may also include wherein the plurality of axially-aligned faces includes 8 faces about a circumference of the locking tube. In addition, this embodiment may also include wherein one or both of the first locking tube and the second locking tube includes at least two diametrical rings. This embodiment may also include wherein the plurality of axially-aligned faces and the plurality of axially-aligned points are arranged in at least one section about a circumference of one or both of the first locking tube and the second locking tube, wherein the at least one section is axially bounded by two of the at least two diametrical rings. This embodiment may also include wherein a length of the at least one section can be the same as or substantially the same as a width of the respective tube clip. Further, this embodiment may also include wherein one of the first and second locking tubes is an offset locking tube.

In another embodiment, a method of using a cluster derotation assembly, the method including securing a first locking tube to a first bone anchor secured to in a vertebra; securing a second locking tube to a second bone anchor secured to in a vertebra; providing a link member having a first tube clip and a second tube clip secured thereto; urging the first tube clip onto the first locking tube until the first locking tube is seated against inner surfaces of a first clip arm and a second clip arm of the first tube clip; directing one or both of the link member and the second tube clip into a position to engage the second locking tube; urging the second tube clip onto a second locking tube until the second locking tube is seated against inner surfaces of a first clip arm and a second clip arm of the second tube clip; securing the first tube clip to a link member; securing the second tube clip to the link member; causing the first and second clip arms of the first tube clip to move from a first position to a second position causing the inner surfaces of the first clip arm and the second clip arm to engage an outer surface of the first locking tube and secure the first tube clip to the first locking tube; causing the first and second clip arms of the second tube clip to move from a first position to a second position causing the inner surfaces of the first clip arm and the second clip arm to engage an outer surface of the second locking tube and secure the second tube clip to the second locking tube; and manipulating at least one of the vertebra using the cluster derotation assembly.

The method may also include wherein the inner surfaces of the first clip arm and the second clip arm of the first tube clip and the inner surfaces of the first clip arm and the second clip arm of the second tube clip include a plurality of axially-aligned ridges and the outer surface of each of the first locking tube and the second locking tube includes a plurality of axially-aligned faces and a plurality of axially-aligned points. Also, the method may further include rotating the first tube clip about the first locking tube to place the second tube clip into a position to engage the second locking tube.

The various parts of the cluster derotation assembly described herein can be made of any suitable material, including metal, plastic, medical grade metals and plastics, injection molded polymers, and the like.

Reference throughout the specification to "various embodiments," "some embodiments," "one embodiment," "some example embodiments," "one example embodiment," or "an embodiment" means that a particular feature, structure, or characteristic described in connection with any embodiment is included in at least one embodiment. Thus, appearances of the phrases "in various embodiments," "in some embodiments," "in one embodiment," "some example embodiments," "one example embodiment, or "in an embodiment" in places throughout the specification are not necessarily all referring to the same embodiment. Furthermore, the particular features, structures or characteristics may be combined in any suitable manner in one or more embodiments.

Following embodiments of the invention are also disclosed:
Embodiment 1: A cluster derotation assembly comprising:
   a. a link member having a surface and an open slot through the link member;
   b. a first tube clip, the first tube clip including a shaft having external threads, a first clip arm extending from a first end of the shaft, and a second clip arm extending from the first end of the shaft, wherein the first and second clip arms are opposed to each other, and wherein the shaft is inserted through the open slot of the link member such that the first and second clip arms of the first tube clip are positioned on a first side of the link member and a second end of the shaft is positioned on a second side of the link member;
   c. a second tube clip, the second tube clip including a shaft having external threads, a first clip arm extending from a first end of the shaft, and a second clip arm extending from the first end of the shaft, wherein the first and second clip arms are opposed to each other, and wherein the shaft is inserted through the open slot of the link member such that the first and second clip arms of the second tube clip are positioned on a first side of the link member and a second end of the shaft is positioned on a second side of the link member;
   d. a first nut with an aperture disposed therein, the aperture including an internal thread that is threadingly engaged to the external thread of the shaft of the first tube clip, wherein when the first nut is tightened onto the threaded shaft of the first tube clip, the contact of the first tube clip with the surface of the link member causes the first and second clip arms to move inward toward a threaded shaft axis;
   e. a second nut with an aperture disposed therein, the aperture including an internal thread that is threadingly engaged to the external thread of the shaft of the second tube clip, wherein when the second nut is tightened onto the threaded shaft of the second tube clip, the contact of the second tube clip with the surface of the link member cause the first and second clip arms to move inward toward a threaded shaft axis.
Embodiment 2: The cluster derotation assembly according to embodiment 1, wherein one or both of the first clip arm and the second clip arm of one or both the first tube clip and the second tube clip comprises an inner surface configured to engage with a complementary surface of a locking tube.
Embodiment 3: The cluster derotation assembly according to embodiment 1 or 2, wherein the inner surface comprises a plurality of axially-aligned ridges and the complementary surface of the locking tube comprises a plurality of axially-aligned faces, wherein intersections of the plurality of axially-aligned faces define a plurality of axially-aligned points.
Embodiment 4: The cluster derotation assembly of one of embodiments 1-3, wherein one or both of the first clip arm and the second clip arm of one or both the first tube clip and the second tube clip are rotatably coupled to the shaft.
Embodiment 5: The cluster derotation assembly of one of embodiments 1-4, wherein one or both of the first tube clip and the second tube clip comprises a knurled washer, wherein an upper surface of an upper portion of the knurled washer is configured to engage the first clip arm and the second clip arm.
Embodiment 6: The cluster derotation assembly of one of embodiments 1-5, wherein one or both of the first tube clip and the second tube clip comprises a wave spring, wherein the wave spring is configured to engage a lower surface of the upper portion of the knurled washer and an upper surface of the link member.
Embodiment 7: The cluster derotation assembly of embodiment 6, wherein each of the upper surface of the link member and a bottom surface of a lower portion of the knurled washer has a machine grooved surface; and wherein the lower portion of the knurled washer extends through an opening defined by the wave spring, such that, when the respective nut is tightened onto the threaded shaft of the respective tube clip, the bottom surface of the lower portion of the knurled washer engages with the upper surface of the link member to reduce or eliminate rotation or translation of the respective tube clip relative to the link member.
Embodiment 8: The cluster derotation assembly of embodiment 6 or 7, wherein a bottom surface of the wave spring is flat or substantially flat.
Embodiment 9: The cluster derotation assembly according to one of embodiments 1-8, wherein one or both of the first nut and second nut comprise wing nuts.
Embodiment 10: The cluster derotation assembly according to one of embodiments 1-9, wherein the first nut comprises a first wing nut and the second nut comprises a second wing nut, wherein the first and second wing nuts nest with each other.
Embodiment 11: A cluster derotation assembly comprising:
   a. a first locking tube having a distal end portion configured to securely join to a bone anchor and a proximal end portion having an outer surface;
   b. a second locking tube having a distal end portion configured to securely join to a bone anchor and a proximal end portion having an outer surface;
   c. a link member having a surface and an open slot through the link member;
   d. a first tube clip having a shaft extending through the open slot, a first clip arm extending from an end of the shaft, and a second clip arm extending from the end of the shaft, wherein one or both of the first clip arm and the second clip arm have an inner surface that is complementary to the outer surface of the proximal end portion of the first locking tube, wherein the first and second clip arms are opposed to each other, and wherein the first and second clip arms are movable between a first position away from a shaft axis and a second position toward a shaft axis such that the inner surface of one or both of the first clip arm and the second clip arm engages the outer surface of the proximal end portion of the first locking tube;
   e. a second tube clip having a shaft extending through the open slot, a first clip arm extending from an end of the shaft, and a second clip arm extending from the end of the shaft, wherein one or both of the first clip arm and the second clip arm have an inner surface that is complementary to the outer surface of the proximal end portion of the second locking tube, wherein the first and second clip arms are opposed to each other, and wherein the first and second clip arms are movable between a first position away from a shaft axis and a second position toward a shaft axis such that the inner surface of one or both of the first clip arm and the second clip arm engages the outer surface of the proximal end portion of the second locking tube.
Embodiment 12: The cluster derotation assembly according to embodiment 11, wherein each of the inner surface of one or both of the first clip arm and the second clip arm of the first tube clip and the inner surface of one or both of the first clip arm and the second clip arm of the second tube clip comprises a plurality of axially-aligned ridges and the outer surface of the proximal end portion for each of the first locking tube and the second locking tube comprises a plurality of axially-aligned faces and a plurality of axially-aligned points.
Embodiment 13: The cluster derotation assembly of embodiment 12, wherein the plurality of axially-aligned faces comprises 8 faces about a circumference of the locking tube.
Embodiment 14: The cluster derotation assembly of one of embodiments 11-13, wherein one or both of the first locking tube and the second locking tube comprises at least two diametrical rings.
Embodiment 15: The cluster derotation assembly of one of embodiments 12-14, wherein the plurality of axially-aligned faces and the plurality of axially-aligned points are arranged in at least one section about a circumference of one or both of the first locking tube and the second locking tube, wherein the at least one section is axially bounded by two of the at least two diametrical rings.
Embodiment 16: The cluster derotation assembly of embodiment 15, wherein a length of the at least one section can be the same as or substantially the same as a width of the respective tube clip.
Embodiment 17: The cluster derotation assembly according to one of embodiments 11-16, wherein one of the first and second locking tubes is an offset locking tube.
Embodiment 18: A method of using a cluster derotation assembly as described in this specification, the method comprising:
   a. securing a first locking tube to a first bone anchor secured to in a vertebra;
   b. securing a second locking tube to a second bone anchor secured to in a vertebra;
   c. providing a link member having a first tube clip and a second tube clip secured thereto;
   d. urging the first tube clip onto the first locking tube until the first locking tube is seated against inner surfaces of a first clip arm and a second clip arm of the first tube clip;
   e. directing one or both of the link member and the second tube clip into a position to engage the second locking tube;
   f. urging the second tube clip onto a second locking tube until the second locking tube is seated against inner surfaces of a first clip arm and a second clip arm of the second tube clip;
   g. securing the first tube clip to a link member;
   h. securing the second tube clip to the link member;
   i. causing the first and second clip arms of the first tube clip to move from a first position to a second position causing the inner surfaces of the first clip arm and the second clip arm to engage an outer surface of the first locking tube and secure the first tube clip to the first locking tube;
   j. causing the first and second clip arms of the second tube clip to move from a first position to a second position causing the inner surfaces of the first clip arm and the second clip arm to engage an outer surface of the second locking tube and secure the second tube clip to the second locking tube; and
   k. manipulating at least one of the vertebra using the cluster derotation assembly.
Embodiment 19: The method according to embodiment 18, wherein the inner surfaces of the first clip arm and the second clip arm of the first tube clip and the inner surfaces of the first clip arm and the second clip arm of the second tube clip comprise a plurality of axially-aligned ridges and the outer surface of each of the first locking tube and the second locking tube comprises a plurality of axially-aligned faces and a plurality of axially-aligned points.
Embodiment 20: The method according to embodiment 18 or 19, further comprising rotating the first tube clip about the first locking tube to place the second tube clip into a position to engage the second locking tube.

## Claims

1. A cluster derotation assembly comprising:
a. a link member (504) having a surface and an open slot (522) through the link member (504);
b. a first tube clip (510), the first tube clip (510) including a shaft (520) having external threads, a first clip arm (528) extending from a first end of the shaft (520), and a second clip arm (530) extending from the first end of the shaft (520), wherein the first and second clip arms (528, 530) are opposed to each other, and wherein the shaft (520) is inserted through the open slot (522) of the link member (504) such that the first and second clip arms (528, 530) of the first tube clip (510) are positioned on a first side of the link member (504) and a second end of the shaft (520) is positioned on a second side of the link member (504);
c. a second tube clip (510), the second tube clip (510) including a shaft (520) having external threads, a first clip arm (528) extending from a first end of the shaft (520), and a second clip arm (530) extending from the first end of the shaft (520), wherein the first and second clip arms (528, 530) are opposed to each other, and wherein the shaft (520) is inserted through the open slot of the link member (504) such that the first and second clip arms (528, 530) of the second tube clip (510) are positioned on a first side of the link member (504) and a second end of the shaft (520) is positioned on a second side of the link member (504);
d. a first nut (512) with an aperture disposed therein, the aperture including an internal thread that is threadingly engaged to the external thread of the shaft (520) of the first tube clip (510), wherein when the first nut is tightened onto the threaded shaft (520) of the first tube clip, the contact of the first tube clip (510) with the surface of the link member (504) causes the first and second clip arms (528, 530) to move inward toward a threaded shaft axis (532);
e. a second nut (513) with an aperture disposed therein, the aperture including an internal thread that is threadingly engaged to the external thread of the shaft (520) of the second tube clip (510), wherein when the second nut (513) is tightened onto the threaded shaft (520) of the second tube clip (510), the contact of the second tube clip (510) with the surface of the link member (504) cause the first and second clip arms (528, 530) to move inward toward a threaded shaft axis (532).

2. The cluster derotation assembly according to claim 1, wherein one or both of the first clip arm (528) and the second clip arm (530) of one or both the first tube clip (510) and the second tube clip (510) comprises an inner surface (542, 544) configured to engage with a complementary surface of a locking tube (502).

3. The cluster derotation assembly according to claim 2, wherein the inner surface (542, 544) comprises a plurality of axially-aligned ridges (586) and the complementary surface of the locking tube (502) comprises a plurality of axially-aligned faces (515), wherein intersections of the plurality of axially-aligned faces (515) define a plurality of axially-aligned points (517).

4. The cluster derotation assembly of one of the preceding claims, wherein one or both of the first clip arm (528) and the second clip arm (530) of one or both the first tube clip (510) and the second tube clip (510) are rotatably coupled to the shaft (520).

5. The cluster derotation assembly of one of the preceding claims, wherein one or both of the first tube clip (510) and the second tube clip (510) comprises a knurled washer (571), wherein an upper surface of an upper portion of the knurled washer (571) is configured to engage the first clip arm (528) and the second clip arm (530); and a wave spring (572), wherein the wave spring (572) is configured to engage a lower surface of the upper portion of the knurled washer (571) and an upper surface of the link member (504).

6. The cluster derotation assembly of claim 5, wherein each of the upper surface of the link member (504) and a bottom surface of a lower portion of the knurled washer (571) has a machine grooved surface; and wherein the lower portion of the knurled washer (571) extends through an opening defined by the wave spring (572), such that, when the respective nut (512, 513) is tightened onto the threaded shaft (520) of the respective tube clip (510), the bottom surface of the lower portion of the knurled washer (571) engages with the upper surface of the link member (504) to reduce or eliminate rotation or translation of the respective tube clip (510) relative to the link member (504).

7. The cluster derotation assembly of claim 5 or 6, wherein a bottom surface of the wave spring (572) is flat or substantially flat.

8. The cluster derotation assembly according to one of the preceding claims, wherein the first nut (512) comprises a first wing nut (512) and the second nut (513) comprises a second wing nut (513), wherein the first and second wing nuts (512, 513) nest with each other.

9. A cluster derotation assembly according to one of the preceding claims, further comprising a first locking tube (502) having a distal end portion configured to securely join to a bone anchor and a proximal end portion having an outer surface; and a second locking tube (502) having a distal end portion configured to securely join to a bone anchor and a proximal end portion having an outer surface; wherein one or both of the first clip arm (528) and the second clip arm (530) of the first tube clip (510) have an inner surface that is complementary to the outer surface of the proximal end portion of the first locking tube (502), and wherein the first and second clip arms (528, 530) of the first tube clip (510) are movable between a first position away from the threaded shaft axis (532) and a second position toward the threaded shaft axis (532) such that the inner surface of one or both of the first clip arm (528) and the second clip arm (530) of the first tube clip (510) engages the outer surface of the proximal end portion of the first locking tube (502); and wherein one or both of the first clip arm (528) and the second clip arm (530) of the second tube clip (510) have an inner surface that is complementary to the outer surface of the proximal end portion of the second locking tube (502), and wherein the first and second clip arms (528, 530) of the second tube clip (510) are movable between a first position away from the threaded shaft axis (532) and a second position toward the threaded shaft axis (532) such that the inner surface of one or both of the first clip arm (528) and the second clip arm (530) of the second tube clip (510) engages the outer surface of the proximal end portion of the second locking tube (502).

10. The cluster derotation assembly according to claim 9, wherein each of the inner surface of one or both of the first clip arm (528) and the second clip arm (530) of the first tube clip (510) and the inner surface of one or both of the first clip arm (528) and the second clip arm (530) of the second tube clip (510) comprises a plurality of axially-aligned ridges (586) and the outer surface of the proximal end portion for each of the first locking tube (502) and the second locking tube (502) comprises a plurality of axially-aligned faces (515) and a plurality of axially-aligned points (517).

11. The cluster derotation assembly of claim 10, wherein the plurality of axially-aligned faces (515) comprises 8 faces about a circumference of the locking tube (502).

12. The cluster derotation assembly of one of claims 9-11, wherein one or both of the first locking tube (502) and the second locking tube (502) comprises at least two diametrical rings (519).

13. The cluster derotation assembly of claim 12, wherein the plurality of axially-aligned faces (515) and the plurality of axially-aligned points (517) are arranged in at least one section about a circumference of one or both of the first locking tube (502) and the second locking tube (502), wherein the at least one section is axially bounded by two of the at least two diametrical rings (519).

14. The cluster derotation assembly of claim 13, wherein a length of the at least one section can be the same as or substantially the same as a width of the respective tube clip (510).

15. The cluster derotation assembly according to one of claims 9-14, wherein one of the first and second locking tubes (502) is an offset locking tube.
